# EUROPÄISCHE PATENTSCHRIFT

(11) **EP 2 440 326 B1**
(45) Veröffentlichungstag und Bekanntmachung des Hinweises auf die Patenterteilung: **04.10.2017**
(21) Anmeldenummer: 10717549.9
(22) Anmeldetag: 23.04.2010
(51) Int. Cl.: B01J 20/24, B01J 20/285, B01J 20/32, B01D 71/16, C08F 251/00, C08F 251/02, C08F 291/08, B01D 15/36, C07K 1/22, C07K 1/18, B01J 39/20, B01D 67/00, C08J 7/14

(54) **POLYSACCHARIDMATRIX MIT AUFGEPFROPFTEM POLYMER, VERFAHREN ZU IHRER HERSTELLUNG UND VERWENDUNG**
POLYSACCHARIDE MATRIX HAVING A GRAFTED POLYMER, METHOD FOR PRODUCING THE SAME AND USE THEREOF
MATRICE DE POLYSACCHARIDES À POLYMÈRE GREFFÉ, SON PROCÉDÉ DE PRODUCTION ET SON UTILISATION

(30) Priorität: 13.06.2009 DE 102009024824; 11.12.2009 DE 102009057993
(43) Veröffentlichungstag der Anmeldung: 18.04.2012
(73) Patentinhaber: Sartorius Stedim Biotech GmbH, 37079 Göttingen (DE)
(72) Erfinder: HÖRL, Hans-Heinrich, 37120 Bovenden (DE); DEMMER, Wolfgang, D-37077 Göttingen (DE); FABER, René, 37077 Göttingen (DE); BRUMM, Christian, 37249 Neu-Eichenberg (DE)
(86) Internationale Anmeldenummer: PCT/EP2010/002496
(87) Internationale Veröffentlichungsnummer: WO 2010/142363

(56) Entgegenhaltungen:
- WO-A1-92/00805
- DE-C2- 2 840 503
- US-A- 4 791 063
- SHUKLA S R ET AL: "GRAFT-COPOLYMERIZATION OF GLYCIDYL METHACRYLATE ONTO COTTON CELLULOSE" JOURNAL OF APPLIED POLYMER SCIENCE, JOHN WILEY AND SONS INC. NEW YORK, US LNKD- DOI:10.1002/APP.1994.070540302, Bd. 54, Nr. 3, 17. Oktober 1994 (1994-10-17), Seiten 279-288, XP000474148 ISSN: 0021-8995
- KAZUHIKO ISHIHARA ET AL: "IMPROVEMENT OF BLOOD COMPATIBILITY ON CELLULOSE DIALYSIS MEMBRANE. \I. GRAFTING OF 2-METHACRYLOYLOXYETHYL PHOSPHORYLCHOLINE ON TO A CELLULOSE MEMBRANE SURFACE" BIOMATERIALS, ELSEVIER SCIENCE PUBLISHERS BV., BARKING, GB LNKD- DOI:10.1016/0142-9612(92)90062-S, Bd. 13, Nr. 3, 1. Januar 1992 (1992-01-01) , Seiten 145-149, XP000258852 ISSN: 0142-9612
- MOSTAFA K M: "Synthesis of poly(acrylamide)-starch and hydrolyzed starch graft copolymers as a size base material for cotton textiles" POLYMER DEGRADATION AND STABILITY, BARKING, GB LNKD- DOI:10.1016/S0141-3910(96)00118-8, Bd. 55, Nr. 2, 1. Januar 1997 (1997-01-01) , Seiten 125-130, XP004294153 ISSN: 0141-3910

## Beschreibung

Die vorliegende Erfindung betrifft eine poröse, nicht partikuläre, konvektiv permeable Polysaccharidmatrix, sowie ein Verfahren zur Herstellung der Polysaccharidmatrix und die Verwendung einer derartigen Polysaccharidmatrix.

Die Filtration, Aufreinigung oder Entfernung von Biomolekülen wie Proteinen, Aminosäuren, Nukleinsäuren, Viren oder Endotoxinen aus flüssigen Medien ist von großem Interesse für die biopharmazeutische Industrie. Poröse, nicht partikuläre Polysaccharidmatrizen, insbesondere in Form von Adsorptionsmembranen, werden vor allem in solchen Prozessen eingesetzt, bei welchen die Adsorbenden in der flüssigen Phase in sehr geringen Konzentrationen in Relation zur Kapazität der Matrix vorliegen, so dass, bezogen auf die Flächeneinheit der Matrix, bis zur Erschöpfung der Kapazität ein großes Volumen der flüssigen Phase verarbeitet werden kann. Eine typische Applikation ist die Adsorption von Desoxy- und Ribonucleinsäuren (RNS und DNS), Viren, Wirtszellproteinen und Endotoxinen, um diese Kontaminanten aus antikörperhaltigen Lösungen mit positiv geladenen Membranen zu entfernen.

Die offene Porenstruktur dieser Membranen erlaubt die Adsorption von großen Adsorbenden im Poreninneren. Bei konventionellen Gelen ist die Adsorption in diesen Fällen auf die äußere Partikeloberfläche limitiert. Daher werden Membranen vor allem bei der Aufreinigung von großen Adsorbenden wie DNS, RNS, Blutfaktor FVIII und Viren erfolgreich eingesetzt. Die kleinere innere Oberfläche der Membranen und die daraus resultierende niedrigere Kapazität ist bei der Adsorption von kleineren Adsorbenden jedoch im Vergleich zu Chromatographiegelen von Nachteil.

Aus DE 39 29 648 C1 und EP 0 490 940 B1 sind Verfahren für die Pfropfung auf stickstoffhaltige Polymere, insbesondere Polyamide, bekannt. Sie beruhen darauf, dass der Wasserstoff von NH-Gruppen der Polymere durch ein Halogen, bevorzugt durch ein Chloratom aus organischen Hypohalogeniten, organischen N-Halogenverbindungen oder Tetrachlormethan substituiert wird, wobei nachfolgend durch Reaktion mit einem Reduktionsmittel eine Stickstoff Radikalbildung erfolgt. Auf die radikalischen Stickstoff-Zentren können dann beliebige ethylenische Monomere aufgepfropft werden. Der Nachteil dieser nach diesen Verfahren hergestellten Membranen, die sich an sich bewährt haben, ist, dass sie deutlich niedrigere hydraulische Permeabilitäten als die Ausgangsmembran aufweisen.

In EP 0 527 992 B1 wird deshalb zur Lösung des Problems der verminderten hydraulischen Permeabilität vorgeschlagen, dass Membranen aus einem ersten Polymer (z. B. Cellulosehydrat, Polyvinylidendifluorid (PVDF) oder Cellulosehydrat) mit einer Lösung eines zweiten Polymers, bevorzugt eines N-chlorierten Nylonderivates, beschichtet werden und nachfolgend mit der so beschichteten Membran eine Aufpfropfung von ethylenischen Monomeren durchgeführt wird. Nachteil dieses Verfahrens ist, daß die ethylenisch ungesättigten Monomere nicht direkt auf das erste Polymer aufgepfropft werden können und die strukturelle Voraussetzung für die Pfropfungsreaktion somit die Beschichtung des ersten Polymers mit der Lösung des zweiten Polymers ist, bevor die Pfropfung erfolgen kann. Dieses Verfahren ist wegen der Vielzahl seiner Einzelschritte kompliziert und kostenintensiv.

Ionenaustauscher auf Basis von hydroxylgruppenhaltigen Trägern (z.B. Fractogel^{®}-TSK der Fa. Merck), auf deren Oberfläche epoxidgruppenhaltige Methacrylsäurederivate aufgepfropft sind, sind aus EP 0 722 360 B1 bekannt. Die Epoxidgruppen des aufgepfropften Polymers können nachfolgend durch NH- oder OH-gruppenhaltige Reagentien zu vicinalen Diol- oder 1,2-Aminoalkoholfunktionen umgesetzt werden.

EP 0 337 144 B1 offenbart hydroxylgruppenhaltige Träger, an deren Oberfläche durch Pfropfpolymerisation herstellbare Polymere kovalent gebunden sind, welche als Wiederholungseinheit die Einheit [-CR'R"-CR¹Y]ₙ mit Y = -CO₂ -CN, -CHO, -OH, -CH₂NH₂ oder -CH₂NR²R³ aufweisen. Die Träger werden zur Fraktionierung von Immunoglobulinen in Humanserum und in Maus-Ascites-Flüssigkeit mit monoklonalen Antikörpern eingesetzt.

EP 1 163 045 B1 offenbart ein Herstellungsverfahren für kationisch modifizierte Membranen, bei welchem eine mikroporöse Ausgangsmembran, bevorzugt aus Polyethersulfon, mit einer Beschichtung versehen wird, wobei die Beschichtung hergestellt wird durch Vernetzen einer Zusammensetzung, welche ein Diallylamin-Copolymer mit Epoxy-Gruppen und kationischen Gruppen, ein Polyalkylenamin und eine aminreaktive Verbindung mit einer kationischen Gruppe umfasst. Bei der aminreaktiven Verbindung handelt es sich bevorzugt um Glycidylverbindungen mit Ammoniumgruppen.

EP 1 614 459 B1 offenbart ein Herstellungsverfahren für kationisch modifizierte Membranen, bei welchem eine mikroporöse Ausgangsmembran, bevorzugt aus (optional hydrophilem) Polyethersulfon, mit einem Gemisch aus einem Copolymer aus Diallylamin, aus einem Diallyldialkylammoniumhalogenid und einem Acrylsäuremonomer mit quartären Ammoniumgruppen behandelt wird und durch Tempern in die kationisch modifzierte Membran überführt wird.

EP 0 538 315 B1 offenbart eine poröse Matrix bestehend aus einem porösen Träger mit Schwammstruktur, der auf seiner inneren und äußeren Oberfläche eine aufgepfropfte Polymerschicht mit funktionellen Gruppen aufweist, wobei die Polymerschicht durch eine mit der Matrix in Kontakt stehende, flüssige Phase derart solvatisierbar ist, daß sie einstellbare Anteile des Porenvolumens des porösen Trägers einnehmen kann. Als Vorläufer für den porösen Träger werden stickstoffhaltige Polymere eingesetzt, z. B. Nylonderivate oder mit Nylonderivaten beschichtetes Cellulosehydrat, auf welche über N-C1-Gruppen als reaktive Zentren Monomere, z. B. in Form eines Gemisches aus Hydroxyethylmethacrylat und Glycidylmethacrylat, aufgepfropft werden.

Y. Chen et al. offenbaren in "Polymer Composites", Bd. 28, 2007, Seiten 47-56, die Herstellung eines Pfropfcopolymers aus Maisstärke und Acrylamid als aufzupfropfendem Monomer in Anwesenheit eines Gemisches aus Cer-(IV)-ammoniumsulfat als Pfropfungsinititator und Zitronensäure. Für eine effiziente Pfropfung auf die Maisstärke ist nach IR-spektroskopischen Untersuchungen der Autoren dabei die oxidative Ringspaltung der Hexose-Bausteine der Maisstärke essentiell, wobei nachfolgend durch Reaktion mit Ce⁴⁺-Kationen radikalische Kohlenstoffatome im Polymerrückgrat der stärke für die Umsetzung mit Acrylamid generiert werden.

EP 1 386 660 B1 offenbart Verfahren zur Isolierung von Immunoglobulinen aus Immunoglobulingemischen im pH-Bereich von 2 bis 10, bei welchen eine Matrix M-SP1-L eingesetzt wird, die jeweils eine Mehrzahl an variablen, funktionellen Gruppen SP1-L aufweist. M ist hierbei eine Chromatographieausgangsmatrix, an welche über Abstandshalter SP1 Liganden L gebunden sind, deren Molekulargewicht höchstens 500 Dalton beträgt. Die Liganden L sind bevorzugt ausgewählt aus der Gruppe der mono-oder bicyclischen (hetero)aromatischen Verbindungen, die optional acide Gruppen tragen können.

EP 0 921 855 B1 offenbart gleichfalls Verfahren zur Isolierung von Immunoglobulinen aus Immunoglobulingemischen im pH-Bereich von 2 bis 10, bei welchen eine Matrix M eingesetzt wird, an welche die funktionelle Gruppe SP1-L gebunden ist. Der Ligand L, welcher durch einen Abstandshalter SP1 an die Matrix M gebunden ist, ist ausgewählt aus der Gruppe der Benzimidazole, Benzothiazole und Benzoxazole. Der Ligand L kann optional an seinem bicyclischen, heteroaromatischen Rest acide Gruppen, wie Sulfon-oder Carbonsäuresubstituenten tragen.

EP 1 718 668 B1 offenbart ein Verfahren zur Abtrennung von Antikörpern von mindestens einer Kontaminante in einer Lösung, bei welchem ein Chromatographieharz verwendet wird, auf welchem ausschließlich multimodale Liganden immobilisiert sind. Multimodale Liganden sind hierbei Liganden, welche mindestens zwei verschiedene (d. h. eine ionogene und eine hydrophobe Interaktion) Bindungswechselwirkungen mit den abzutrennenden Komponenten eingehen können. Die Liganden weisen für diese beiden Bindungswechselwirkungen kationenaustauschende Gruppen und zumindest ein (hetero)aromatisches Ringsystem auf.

US 4 791 063 offenbart eine Polysaccharidmatrix in Form von Cellulose-Filterpads, welche mit Acrylatmonomeren dergestalt modifiziert werden, dass sie Epoxidgruppen enthalten. Diese Epoxidgruppen werden durch Oxidation in anionische Gruppen umgewandelt, so dass ein zum Kationenaustausch befähigtes Material erhalten wird, welches zur Stofftrennung, nämlich der chromatographischen Adsorption von bovinem gamma-Globulin, verwendet wird. Der vorliegenden Erfindung liegt die Aufgabe zugrunde, eine poröse, nicht partikuläre, konvektiv permeable Polysaccharidmatrix bereitzustellen, auf deren Oberfläche ein aufgepfropftes Polymer fixiert ist und die sich durch eine hohe Proteinbindungskapazität auszeichnet, sowie kostengünstige und effiziente Verfahren zur Herstellung dieser Polysaccharidmatrix bereitzustellen. Ferner soll eine Verwendung der Polysaccharidmatrix zur Stofftrennung vorgeschlagen werden.

Diese Aufgabe wird gelöst durch die erfindungsgemäße Polysaccharidmatrix nach Anspruch 1, die Verfahren zu ihrer Herstellung nach den Ansprüchen 8 und 9 und durch die Verwendung der erfindungsgemäßen Membran nach Anspruch 12 zur Stofftrennung.

Im Rahmen der vorliegenden Erfindung werden unter einer porösen, nicht partikulären Polysaccharidmatrix offenporige Schäume oder mikroporöse Membranen, die jeweils ein Polysaccharid umfassen, verstanden. Bevorzugt sind mikroporöse Membranen mit einer schwammartigen Struktur, die aus einem zusammenhängenden räumlichen System von Poren bestehen und daher im Verhältnis zu ihrem Strömungswiderstand eine hohe spezifische Oberfläche aufweisen. Die typische Schichtdicke der als Polysaccharidmatrix verwendbaren mikroporösen Membranen liegt im Bereich zwischen 50 und 500 µm, vorzugsweise zwischen 100 und 300 µm. Es sind mittlere Porengrößen im Bereich von 0,01 bis 20 µm bevorzugt, besonders bevorzugt von 0,1 bis 15 µm und am meisten bevorzugt im Bereich von 0,4 bis 10 µm, wobei die mittlere Porengröße mit einem Coulter-Porometer des Typs "Capillary Flow Porometer 6.0", CAPWIN Software System, Fa. Porous Materials Inc., bestimmt wird. Derartige Membranen können eben oder zylindrisch ausgebildet sein. Zylindrische Membranen werden als Membranhohlfasern, Membrankapillaren oder Membranschläuche bezeichnet. Bevorzugt im Rahmen der Erfindung sind Flachmembranen, wobei in Wickel- oder Stapelmodule integrierte Adsorptionsmembranen besonders bevorzugt sind.

Im Rahmen der vorliegenden Erfindung soll unter einer konvektiv permeablen Polysaccharidmatrix eine Polysaccharidmatrix verstanden werden, bei welcher durch Einwirken einer hydraulischen Druckdifferenz eine konvektive Durchströmung mit einem Adsorbenden enthaltenden Medium erreicht wird, wodurch anstelle eines rein diffusen Stofftransports des oder der Adsorbenden in Richtung eines Konzentrationsgradienten ins Innere der Matrix ein konvektiver Stofftransport erreicht wird, der bei hohem Durchfluss sehr viel rascher als ein diffusiver Stofftransport erfolgt.

Dadurch kann ein den partikulären Matrizen (z. B. Chromatographiegelen) inhärenter Nachteil, der als Diffusionslimitierung bezeichnet wird, vermieden werden. Dieser Nachteil besteht darin, dass mit zunehmender Partikelgröße der Matrix und mit zunehmender Molmasse des Adsorbenden die erforderliche Zeit zur Einstellung des Adsorptionsgleichgewichts erheblich zunimmt, was sich in einer Verschlechterung der Kinetik auswirkt.

Bei einer bevorzugten Ausführungsform besteht die Polysaccharid-Ausgangsmatrix aus Polysaccharid, während sie bei einer weiteren Ausfiihrungsform ein poröser Träger mit einer Polysaccharidbeschichtung ist.

Bei diesen beiden Ausführungsformen ist das Polysaccharid ausgewählt aus der Gruppe, bestehend aus Celluloseestern, Celluloseethern, Cellulosehydrat, Agarose, Chitin, Chitosan, Dextran oder Kombinationen davon. Optional können die vorgenannten Polysaccharide vernetzt sein.

Besonders bevorzuge Polysaccharide sind Celluloseester, insbesondere Cellulosemonoacetat, Cellulosediacetat, Cellulosetriacetat, Cellulosepropionat, Cellulosebutyrat, Celluloseacetobutyrat oder Cellulosenitrat, Celluloseether, insbesondere Methylcellulose oder Ethylcellulose, sowie Gemische davon, wobei Celluloseacetate, insbesondere Cellulosediacetat, am meisten bevorzugt sind.

Bei der vorgenannten Ausfiihrungsform eines porösen Trägers mit einer Polysaccharidbeschichtung ist der poröse Träger bevorzugt mikroporös und besteht aus einem Polymer, welches ausgewählt ist aus der Gruppe, bestehend aus Polyamid, Poly(ether)sulfon, Polyvinylidendifluorid, Polyacrylnitril, Polyvinylchlorid, Polypropen, Polyethen, Polytetrafluorethen, deren Copolymeren oder Gemischen davon.

Auf der Oberfläche der erfindungsgemäßen, porösen Polysaccharidmatrix ist ein aufgepfropftes Polymer aus mindestens einer ethylenischen Monomerverbindung mit Epoxid-Gruppen fixiert, wobei unter Oberfläche der porösen Matrix sowohl die innere Oberfläche, d. h. die Poreninnenwandflächen, als auch die äußere Oberfläche, d. h. die äußeren Flächen der porösen Matrix, verstanden werden.

Bevorzugt ist das auf die Polysaccharid-Ausgangsmatrix aufgepfropfte Polymer aus mindestens einer ethylenischen Monomerverbindung gebildet, welche ein (Meth)Acrylsäurederivat ist und deren funktionelle Gruppen Epoxid-Gruppen sind.

Im Sinne der vorliegenden Erfindung sind besonders ethylenische Monomerverbindungen, ausgewählt aus der Gruppe bestehend aus Styrol, alpha-Methylstyrol, 4-Ethoxystyrol, 3,4-Dimethoxystyrol, 4-Benzyloxy-3-methoxystyrol, Fluorstyrol, Chlormethylstyrol, 4-tert-Butylstyrol, Vinylanisol, 4-Vinylbenzoesäure, 4-Vinylbenzylchlorid, Vinylanilin, N,N-Dimethylvinylbenzylamin, Vinylbenzyltrimethylammoniumchlorid, 4-Vinylbiphenyl, 2-Vinylnaphthalin, N-(Isobutoxymethyl)acrylamid, N-(Butoxymethyl)acrylamid, N-tert-Butylacrylamid, N,N-Dimethylacrylamid, N-Isopropylacrylamid, N-Phenylacrylamid, N-[Tris(hydroxymethyl)methyl]acrylamid, Benzyl-2-propylacrylat, Ethylenglycolphenyletheracrylat, Hexylacrylat, Isooctylacrylat, Tetrahydrofurylacrylat, 2,2,3,3,4,4,5,5,6,6,7,7-Dodecafluorheptylacrylat, Benzylmethacrylat, Butylmethacrylat, 3-Chlor-2-hydroxypropylmethacrylat, Cyclohexylmethacrylat, Ethylenglycolphenylethermethacrylat, 2-(Methyl-thio)ethylmethacrylat, Phenylmethacrylat, Poly(propylenglycol)methacrylat, Allylbenzylether, Vinylpyridin, Vinylacetat, [Poly(ethylenglycol)₍ₙ₎]monomethacrylat oder Kombinationen davon, bevorzugt. Diese vorgenannten Monomere weisen als funktionelle Gruppen, die auch das auf die Polysaccharidmatrix aufgepfropfte Polymer aufweist, hydrophobe Gruppen auf.

Bei einer weiteren bevorzugten Ausführungsform der erfindungsgemäßen Membran wird für ihre Herstellung mindestens eine ethylenische Monomerverbindung verwendet, welche als funktionelle Gruppen ionogene bzw. in Ionen überführbare Gruppen, bevorzugt Säuregruppen, umfaßt. Bevorzugt sind insbesondere Acrylsäure, Methacrylsäure, 2-Acrylamidoglycolsäure, 2-Acrylamido-2-methyl-1-propansulfonsäure, (3-Acrylamidopropyl)trimethylammoniumchlorid, N,N-Dimethylacrylamid, N-[3-(Dimethylamino)propyl]methacrylamid, [2-(Acryloyloxy)ethyl]trimethylammoniumchlorid, Diallyldimethylammoniumchlorid, 2-Carboxyethylacrylat, 2-(Dimethylamino)ethylacrylat, 2-Aminoethylmethacrylat, 2-(Diethylamino)ethylmethacrylat, [3-(Methacryloylamino)propyl]dimethyl(3-sulfopropyl)ammoniumhydroxid, [3-(Methacryloylamino)propyl]trimethylammoniumchlorid, [3-(Acryloylamino)propyl]trimethylammoniumchlorid [2-(Methacryloyloxy)ethyl]trimethylammoniumchlorid, 1-(3-Sulfopropyl)-2-vinylpyridiniumhydroxid, Sulfoethyl(meth)acrylat, Sulfopropyl-(meth)acrylat, Vinylsulfonsäurederivate, Styrolsulfonsäurederivate oder Kombinationen davon.

Bei einer weiteren Ausführungsform ist die erfindungsgemäße Membran hergestellt unter Verwendung von mindestens einer ethylenischen Monomerverbindung, welche als funktionelle Gruppen polare, nicht ionogene Gruppen umfasst, insbesondere Hydroxypropyl(meth)acrylat, 3-(Meth)Acryloylamino-1-propanol, 4-Hydroxybutyl(meth)acrylat, 2-Hydroxy-3-phenoxypropylacrylat, 2-Hydroxyethyl(meth)acrylat, Glycerolmonomethacrylat, Vinylpyrrolidon, Vinylalkohol oder Kombinationen davon. Die ethylenische Monomerverbindung im Sinne der vorliegenden Erfindung umfaßt als funktionelle Gruppen Epoxidgruppen, die nachfolgend mit Nucleophilen umgesetzt werden können. Besonders bevorzugt sind Glycidylacrylat und Glycidylmethacrylat.

Bei einer weiteren Ausführungsform ist die Membran hergestellt aus mindestens einer ethylenischen Monomerverbindung, welche als funktionelle Gruppen vernetzungsfähige Gruppen umfaßt, die nach Aufpfropfen des ethylenischen Monomers auf die Ausgangsmatrix eine Vernetzung des aufgepfropften Polymers ermöglichen, z. B. Ethylenglycoldimethacrylat, Glycerindimethacrylat, 1,4-Butandioldiacrylat, Diethylenglycoldimethacrylat, Tetraethylenglycoldimethacrylat, [Poly(ethylenglycol)₍ₙ)]diacrylat, [Poly(ethylenglycol)₍ₙ₎]dimethacrylat, 1,3-Diisopropenylbenzol, Bisphenol-A-dimethacrylat, Allylmethacrylat, 3-(Acryloyloxy)-2-hydroxypropyl-methacrylat, 1,3-Butandioldiacrylat, 1,4-Butandioldiacrylat, 1,4-Butandioldimethacrylat, N,N'-Methylen-bis(acrylamid), 1,3,5-Triacryloylhexahydro-1,3,5-triazin, 1,1,1 -Trimethylolpropantriacrylat, Poly(ethylenglycol)-bisphenol-A-diglycidylether-Addukte oder Kombinationen davon. Diese Monomere mit vernetzungsfähigen Gruppen können entweder allein oder in Kombination mit mindestens einer der vorgenannten Monomerverbindungen zur Herstellung einer Membran eingesetzt werden.
Weiterhin können alle vorgenannten Typen von Monomerverbindungen jeweils allein oder in Kombination mit mindestens einer weiteren, beliebigen Sorte der vorgenannten Monomerverbindungen auf die Polysaccharid-Ausgangsmatrix aufgepfropft werden.

In einer besonders bevorzugten Ausführungsform ist die erfindungsgemäße Polysaccharidmatrix durch Pfropfen eines Gemisches von Glycidylacrylat oder Glycidylmethacrylat mit Benzylmethacrylat hergestellt.

Das Pfropfen der vorgenannten mindestens einen ethylenischen Monomerverbindung auf die Polysaccharid-Ausgangsmatrix erfolgt dabei erfindungsgemäß in Anwesenheit einer Übergangsmetall- oder Lanthanoidverbindung, welche radikalische

Zentren auf der Ausgangsmatrix erzeugt, auf welche die mindestens eine ethylenische Monomerverbindung radikalisch aufgepfropft wird. Erfindungsgemäß wird die
Membran hergestellt durch Pfropfen in Anwesenheit einer
Übergangsmetall- oder Lanthanoidverbindung aus der Gruppe der Cer-, Mangan-,
Eisen-, Nickel-, Cobalt-, Vanadiumverbindungen oder Kombinationen davon.
Besonders bevorzugt werden Ce-(IV)-Salze eingesetzt.

Überraschenderweise wurde gefunden, daß die erfindungsgemäße Polysaccharidmatrix
eine signifikant erhöhte Proteinbindungskapazität aufweist, wenn das Pfropfen der mindestens einen ethylenischen Monomerverbindung auf die Polysaccharid-Ausgangsmatrix in Anwesenheit einer organischen Säure wie unten beschrieben
erfolgt. Insbesondere die statische Bindungskapazität für Rinderserumalbumin
(RSA) kann bis um das 8,4fache gesteigert werden in Bezug auf eine
Vergleichspolysaccharidmatrix, bei der alle Herstellungsparameter bis auf den Einsatz dieser vorgenannten organischen Säure identisch sind.

Unter statischer Bindungskapazität soll hierbei die im Gleichgewicht gebundene Masse eines Adsorbenden (z. B. RSA) bezogen auf die Flächeneinheit einer besonders bevorzugt als Membran vorliegenden, erfindungsgemäßen Polysaccharidmatrix in mg/cm² verstanden werden.

Die erfindungsgemäßen Polysaccharidmatrizen mit gegenüber der vorstehend genannten Vergleichsmatrix um das 1,5- bis 8,4-fache erhöhten statischen

Proteinbindungskapazitäten sind durch das vorstehend beschriebene Pfropfen in Anwesenheit einer organischen Säure herstellbar, wobei diese organische Säure ausgewählt ist aus der Gruppe bestehend aus Citronensäure, Äpfelsäure, Weinsäure, Fumarsäure, Maleinsäure, Bernsteinsäure, Oxalsäure, Malonsäure, Ascorbinsäure, Glucuronsäure, Milchsäure oder Kombinationen davon. Gemäß einer Ausführungsform der vorliegenden Erfindung können die funktionellen Gruppen des auf die Polysaccharid-Ausgangsmatrix gepfropften Polymers per se zur Wechselwirkung mit in Fluiden enthaltenen Adsorbenden befähigt sein. Gemäß der Erfindung werden nach dem Pfropfen die funktionellen Gruppen des auf die Polysaccharid-Ausgangsmatrix gepfropften Polymers mit mindestens einem Liganden, der zur Wechselwirkung mit in Fluiden enthaltenen Adsorbenden befähigt ist, umgesetzt. Erfindungsgemäß weist der Ligand kationische und/oder anionische Gruppen auf. Der vorgenannte Ligand reagiert mit den funktionellen Gruppen der Monomereinheiten des aufgepfropften Polymers, wodurch jede Monomereinheit in eine durch den mindestens einen Liganden funktionalisierte Wiederholungseinheit des aufgepfropften Polymers transformierbar ist ("Polymeranaloge Reaktion" des Liganden).

Als Ligand mit kationischer Gruppe bevorzugt im Rahmen dieser Erfindung sind primäre Amine mit einem Stickstoffatom, insbesondere ausgewählt aus der Gruppe bestehend aus Butylamin, Hexylamin, Cyclopropylamin, Cyclohexylamin, Anilin, Chloranilin, Benzylamin, Phenylethylamin, Phenylpropylamin, Phenylhexylamin, 2,3,4,5,6-Pentafluoranilin, 4-(Trifluormethyl)anilin, Toluidin, 1-(4-Methylphenyl)ethylamin, 1-Naphthylamin, 1-Methyl-3-phenylpropylamin oder Kombinationen davon, sekundäre Amine mit einem Stickstoffatom, wie z.B. Dimethylamin, N-Ethylmethylamin, Diisopropylamin, N-Methylanilin, N-Methylcyclohexylamin, 4-Trifluormethyl-N-methylanilin, N-Benzylmethylamin, N-Ethylanilin, N-Methylethanolamin, 4-Methoxy-N-methylanilin, N-Ethylcyclohexylamin, N-Ethylbenzylamin, 2-Methoxy-N-methylbenzylamin, Diphenylamin, N-Cyclohexylanilin, Dibenzylamin oder Kombinationen davon sowie tertiäre Amine mit einem Stickstoffatom ausgewählt aus der Gruppe bestehend aus Trimethylamin, N,N-Dimethylethylamin, N,N-Diethylmethylamin, N,N-Dimethylbutylamin, N,N-Dimethyl-hexylamin, N-Methyldipropylamin, 6-(Dimethylamino)fulven, N,N-Dimethylanilin, N,N-Dimethylcyclohexylamin, N,N-Dimethylbenzylamin, N,N-Dimethyl-1-naphthylamin, N-Hexylanilin, Dicyclohexylamin, N-Benzylanilin, N,N-Dicyclo-hexylmethylamin, N-Benzyl-N-ethylanilin, N-Benzyl-N-hydroxyethyl-N-methylamin oder Kombinationen davon.

Eine weitere im Sinne dieser Erfindung bevorzugte Gruppe von Liganden mit mehr als einer kationischen Gruppe sind Amine mit mehr als einem Stickstoffatom, welche nachfolgend als Polyamine bezeichnet werden. Durch Umsetzen der funktionellen Gruppen des auf die Polysaccharid-Ausgangsmatrix aufgepfropften Polymers mit den vorgenannten Polyaminen kann das aufgepfropfte Polymer vernetzt werden und die Dichte positiver Ladungsträger durch Quaternisierung der Stickstoffatome erhöht werden. Besonders bevorzugt sind Polyamine ausgewählt aus der Gruppe bestehend aus Ethylendiamin, 1,4-Diaminobutan, 1,6-Diaminohexan, 1,8-Diaminooctan, N,N-Dimethylethylendiamin, 3-(Dimethylamino)-1-propylamin, Diethylentriamin, N,N'-Dimethyl-1,3-propandiamin, N,N,N'-Trimethylethylendiamin, 2-(Aminomethyl)-2-methyl-1,3-propandiamin, 1,4-Phenylendiamin, Hexamethylentetramin, N,N-Diethylethylendiamin, N,N,N',N'-Tetramethylethylendiamin, Diethyldimethylpropandiamin, N,N,N'-Trimethyl-1,3-propandiamin, Triethylentetramin, Tris(2-aminoethyl)amin, 4-Aminobenzylamin, N,N,N',N'-Tetramethyl-l,3-propandiamin, 3,3'-Diamino-N-methyldipropylamin, Tris(dimethylamino)methan, 4-(2-Aminoethyl)anilin, N,N-Dimethyl-p-phenylendiamin, N-Phenylethylendiamin, N,N'-Dimethyl-1,6-hexandiamin, Bis[2-(N,N-dimethylamino)ethyl] ether, Tetraethylenpentamin, Polyethylenimine variabler Molekulargewichte zwischen 500 und 500.000 g/mol, N-Benzylethylendiamin, N-Cyclohexyl-1,3-propandiamin, N,N,N',N",N"-Pentamethyldiethylentriamin, N,N,N',N'-Tetramethyl-p-phenylendiamin, 1,4-Bis(3-aminopropoxy)butan, 4,4'-Oxydianilin, 2-[2-(Dimethylaminoethyl)-methylamino]ethanol, Piperazin, N-Methylpiperazin, N,N-Dimethylpiperazin, Aminoethylpiperazin, 1,4-Bis-(3-aminopropyl)-piperazin, 1-(2-Hydroxyethyl)piperazin, Diazabicyclooctan, Agmatin, S-Triazin oder Kombinationen davon.

Bei Verwendung der vorgenannten Liganden mit einem oder mehr als einem Stickstoffatom wird die erfindungsgemäße Polysaccharidmatrix durch Pfropfen mindestens einer ethylenischen Monomerverbindung hergestellt, welche als funktionelle Gruppen Epoxidgruppen aufweist, welche durch Umsetzung mit dem oder den Stickstoffatom(en) des Liganden eine Ringöffnungsreaktion eingehen. Besonders bevorzugt als ethylenische Monomerverbindungen sind bei dieser Ausführungsform Glycidylacrylat und/oder Glycidylmethacrylat.

Bei Liganden mit mehr als einem Stickstoffatom kann durch die Ringöffnungsreaktion der Epoxidgruppen durch mehrere dieser Stickstoffatome das aufgepfropfte Polymer der erfindungsgemäßen Polysaccharidmatrix vernetzt werden, wobei diese Vernetzung intermolekular mit Epoxidgruppen zweier verschiedener aufgepfropfter Polymerketten oder intramolekular mit Epoxidgruppen derselben aufgepfropften Polymerkette erfolgen kann.

Bei einer weiteren Variante der Erfindung, bei welcher Polyamine mit mehr als einem Stickstoffatom, besonders bevorzugt mit zwei Stickstoffatomen, als Ligand eingesetzt werden, erfolgt eine Ringöffnungsreaktion der Epoxidgruppen durch nur eines dieser Stickstoffatome, während das oder die nicht an der Ringöffnungsreaktion beteiligten, übrige(n) Stickstoffatom(e) zu einer Erhöhung der Dichte von ionenaustauschenden Gruppen auf der erfindungsgemäßen Polysaccharidmatrix beiträgt bzw. beitragen.

Besonders bevorzugt ist das als Ligand eingesetzte Polyamin ein symmetrisches oder asymmetrisches Polyamin der Formel (I)

**R¹R²N-B-NR³R⁴** **(I),**

wobei über die Länge und Struktur der Brücke B steuerbar ist, ob nur die Gruppe R¹R²N oder auch die Gruppe NR³R⁴ eine Ringöffnungsreaktion mit Epoxidgruppen des aufgepfropften Polymers eingeht.
Bei symmetrischen Polyaminen sind die Gruppen R¹R²N und NR³R⁴ gleich und die Brücke B weist eine symmetrische Struktur auf, d. h. die Brücke B weist eine Spiegelebene senkrecht zu einer gedachten, durch die cyclische oder acyclische Molekülkette der Formel (I) verlaufenden Achse auf. Die Reaktivität der beiden Amino-Gruppen ist gleich, wodurch die Wahrscheinlichkeit der zweifachen Ringöffnungsreaktion der Epoxidgruppen durch die R¹R²N- und die Gruppe NR³R⁴ erhöht ist. Die Reaktion über nur eine dieser beiden Amino-Gruppen kann durch die Länge der Brücke B und die Konzentration des Diamins in der verwendeten Reaktionsflotte beeinflusst werden. Je kürzer die Brücke B und je höher die Konzentration des Diamins in der Flotte ist, desto höher ist die Wahrscheinlichkeit einer Ringöffnungsreaktion durch nur eine der Gruppen R¹R²N oder NR³R⁴. Alternativ kann eine dieser Aminogruppen durch geeignete Schutzgruppen vor der Reaktion mit funktionellen Gruppen des aufgepfropften Polymers geschützt werden. Die Methoden der Schutzgruppeneinfuhrung in Amine sind dem Fachman bekannt ("Protective Groups in Organic Synthesis", dritte Auflage, Theodora W. Greene, Peter G.M. Wuts, 1999).

Beispiele für im Sinne der Erfindung bevorzugte, symmetrische Polyamine sind z.B. N,N,N',N'-Tetramethylethylendiamin, N,N,N',N'-Tetramethyl-1,3-propandiamin, Tetraethylenpentamin, Pentaethylenhexamin, N,N,N',N",N"-Pentamethyldiethylentriamin, Bis[2-(N,N-dimethylamino)ethyl] ether, Tetramethyl-1,6-hexandiamin, N,N'-Dimethyl-piperazin, 1,2-Diaminocyclohexan, ortho- bzw. para-Xylylendiamin, N,N,N',N'-Tetramethyl-l,4-butandiamin oder 1,4-Diazabicyclo[2.2.2]octan.

Bei asymmetrischen Polyaminen sind die Substituenten der R¹R²N- und der NR³R⁴-Gruppe unterschiedlich. Hieraus resultiert eine unterschiedliche Reaktivität der beiden Aminogruppen. Dadurch läuft die Reaktion nur einer Aminogruppe R¹R²N- oder N R³R⁴- mit dem aufgepfropften Polymer bevorzugt ab.
Beispiele für asymmetrische Diamine sind z.B. 3-(Dimethylamino)-1-propylamin, N,N,N'-Trimethyl-1,3-propandiamin, 2-Amino-5-diethylaminopentan, N,N-Diethyl-N', N'-dimethyl-1,3-propandiamin, N,N-Diethyl-N',N'-dimethyl-1,2-propandiamin, 2-{[2-(Dimethylamino)ethyl]methylamino}ethanol, 1-(2-Hydroxyethyl)piperazin oder Kombinationen davon.

Bei einer weiteren Ausführungsform der vorliegenden Erfindung wird ein Ligand eingesetzt, welcher mindestens eine anionische Gruppe aufweist. Besonders bevorzugt sind Liganden mit Sulfonsäuregruppen. Diese Sulfonsäure-Liganden sind z. B. durch Reaktion von SO₃2⁻-Anionen oder deren Derivaten mit den funktionellen Gruppen der Monomereinheiten des aufgepfropften Polymers in die erfindungsgemäße Membran einführbar, wodurch ein Teil oder alle Monomereinheiten in eine durch eine Sulfonsäure-Gruppe (SO₃H-Gruppe funktionalisierte Wiederholungseinheit des aufgepfropften Polymers transformierbar ist bzw. sind. In diesem Fall ist die funktionelle Gruppe der aufgepfropften Monomereinheit erfindungsgemäß eine Epoxid-Gruppe. Besonders bevorzugt ist die ethylenische Monomerverbindung Glycidylmethacrylat.

Gemäß einer weiteren bevorzugten Variante können nach dem Pfropfen die funktionellen Gruppen des auf die Polysaccharid-Ausgangsmatrix gepfropften Polymers mit zwei verschiedenen Liganden umgesetzt werden, von denen der erste Ligand eine anionische und der zweite Ligand eine hydrophobe Gruppe aufweist.

In diesem Fall sind die funktionellen Gruppen des aufgepfropften Polymers erfindungsgemäß Epoxid-Gruppen, welche mit den zwei verschiedenen Liganden in einer Ringöffnungsreaktion umgesetzt werden. Besonders bevorzugt ist bei dieser Ausführungsform das aufgepfropfte Polymer aus Glycidylmethacrylat gebildet.

Besonders bevorzugt handelt es sich bei dem ersten Liganden um die Sulfonsäuregruppe -SO₃H, die wie vorstehend beschrieben ausgehend von SO₃²⁻-Ionen oder deren Derivaten in die Membran einführbar ist, und bei dem zweiten Liganden um Anilin oder Sulfanilsäure (4-Aminobenzolsulfonsäure) mit einem hydrophoben Arylrest.

Am meisten bevorzugt handelt es sich bei dem ersten Liganden um die Sulfonsäuregruppe und bei dem zweiten Liganden um Sulfanilsäure.

Bei erfindungsgemäßen Membranen mit dieser vorgenannten Ligandenkombination aus Sulfonsäure- und Sulfanilsäuregruppe wurde überraschenderweise festgestellt, daß sie auch bei Salzkonzentrationen bis 300 mM NaCl eine hohe Proteinbindungskapazität aufweisen, wohingegen Vergleichsmembranen, die unter sonst gleichen Bedingungen hergestellt wurden, aber nur Sulfanilsäure als einzigen Liganden aufweisen, im Bereich von Salzkonzentrationen von 150 bis 300 mM NaCl praktisch keine Proteinbindungskapazität aufweisen. Diese Ausführungsform der erfindungsgemäßen Membran mit Sulfonsäure- und Sulfanilsäureligand ist deshalb insbesondere als Kationenaustauscher zur Kontaminantenentfernung und Anreicherung von Biomolekülen geeignet, wobei in derartigen Verfahren Kationenaustauscher wegen ihrer hohen Salzempfindlichkeit bisher nicht einsetzbar waren. Unter Adsorbenden, mit welchen der mindestens eine Ligand zu einer Wechselwirkung befähigt ist, sollen im Rahmen dieser Erfindung eine oder mehrere Zielsubstanzen und/oder Kontaminanten biotechnologischer Prozesse verstanden werden. Die Adsorbenden können Einzelmoleküle, Assoziate oder Partikel sein, bei denen es sich erfindungsgemäß um Proteine, Nucleinsäuren, Viren, monoklonale Antikörper oder andere Substanzen biologischen Ursprungs handelt.

Die Wechselwirkung der Adsorbenden mit den an die erfindungsgemäße Polysaccharidmatrix gebundenen Liganden kann reversibel oder irreversibel sein, in jedem Fall ermöglicht sie die Abtrennung der Adsorbenden von Fluiden, bei denen es sich bevorzugt um wässrige Flüssigkeiten handelt. Zielsubstanzen sind Wertstoffe, die aus dem Fluid in angereicherter oder reiner Form gewonnen werden sollen. Kontaminanten sind Stoffe, deren Entfernung aus dem Fluid aus technischen, regulatorischen oder sonstigen Gründen erforderlich oder wünschenswert ist. Für die Entfernung von Kontaminanten, die als "negative Adsorption" bezeichnet wird, kann (darf) die Adsorption irreversibel verlaufen, wenn die Polysaccharidmatrix nur einmal verwendet werden soll. Bei der Adsorption der Zielsubstanz(en) muss der Vorgang reversibel verlaufen. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können.

Die erfindungsgemäßen Polysaccharidmatrizen werden bevorzugt nach zwei alternativen Verfahren hergestellt, in deren Verlauf als gemeinsames Merkmal das Pfropfen der mindestens einen ethylenischen Monomerverbindung in Anwesenheit der Übergangsmetall- oder Lanthanoidverbindung und in Anwesenheit der organischen Säure erfolgt.

Das erste Verfahren zur Herstellung der erfindungsgemäßen Polysaccharidmatrix ist das im Anspruch 8 definierte Herstellungsverfahren. Erfindungsgemäß wird in Schritt A) eine mit der organischen Säure behandelte Polysaccharid-Ausgangsmatrix bereitgestellt, indem die organische Säure wahlweise bereits der Gießlösung für die Herstellung der Ausgangsmatrix zugesetzt wird oder die im Wesentlichen gesamte Oberfläche der porösen Ausgangsmatrix nach ihrer Herstellung mit der organischen Säure in Kontakt gebracht wird. Vorzugsweise erfolgt dieses Inkontaktbringen durch Eintauchen, Besprühen oder Imprägnieren der Ausgangsmatrix mit einer Lösung der organischen Säure in einem geeigneten Lösungsmittel, besonders bevorzugt Wasser oder Gemische aus Wasser und organischen Lösungsmitteln. Die Konzentration der organischen Säure bezogen auf die Polysaccharid-Ausgangsmatrix beträgt 0,03 bis 300 mmol Säure/kg Ausgangsmatrix, bevorzugt 0,1 bis 100 mmol Säure/kg Ausgangsmatrix und am meisten bevorzugt 0,2 bis 20 mmol Säure/kg Ausgangsmatrix.

Das nachfolgende Pfropfen in Schritt B) erfolgt bevorzugt mit einer Pfropfflotte, umfassend die Übergangsmetall- oder Lanthanoidverbindung, die mindestens eine ethylenische Monomerverbindung und Wasser oder mindestens ein organisches Lösungs- oder Emulsionsmittel bzw. Gemische davon. Die Pfropfflotte kann als Lösung oder Emulsion vorliegen. Die Konzentration der Übergangsmetall- oder Lanthanoidverbindung in der Pfropfflotte beträgt 0,1 bis 100 mmol/l, bevorzugt 0,5 bis 50 mmol/l und besonders bevorzugt 2 bis 30 mmol/l. Die Konzentration der mindestens einen ethylenischen Monomerverbindung in der Pfropfflotte beträgt unter 50 Gew%, bevorzugt unter 20 Gew% und am meisten bevorzugt unter 5 Gew%.

Gemäß einer Variante dieses ersten Verfahrens zur Herstellung der erfindungsgemäßen Polysaccharidmatrix wird in Schritt A) eine poröse, mit der organischen Säure behandelte Polysaccharid-Ausgangsmatrix bereitgestellt und nachfolgend Schritt B) mit einer die Monomerverbindung, die Übergangsmetall- oder Lanthanoidverbindung und zusätzlich die organische Säure umfassenden Pfropfflotte durchgeführt. Die Konzentration der organischen Säure bezogen auf die Polysaccharid-Ausgangsmatrix beträgt 0,01 bis 300 mmol Säure/kg Ausgangsmatrix, bevorzugt 0,2 bis 100 mmol Säure/kg Ausgangsmatrix und am meisten bevorzugt 0,3 bis 20 mmol Säure/kg Ausgangsmatrix, während die Konzentration der organischen Säure in der Pfropfflotte zwischen 0,01 bis 100 mmol/l, bevorzugt zwischen 0,05 bis 30 mmol/l und am meisten bevorzugt zwischen 0,1 und 3 mmol/l beträgt.

Die Konzentration der Übergangsmetall- oder Lanthanoidverbindung in der Pfropfflotte beträgt bei dieser Variante 0,1 bis 100 mmol/l, bevorzugt 0,5 bis 50 mmol/l und besonders bevorzugt 2 bis 30 mmol/l.

Das zweite Verfahren zur Herstellung der erfindungsgemäßen Polysaccharidmatrizen ist das im Anspruch 9 definierte Herstellungsverfahren. Erfindungsgemäß wird in Schritt A) eine mit der Übergangsmetall- oder Lanthanoidverbindung behandelte Polysaccharid-Ausgangsmatrix bereitgestellt, indem die im Wesentlichen gesamte Oberfläche der porösen Ausgangsmatrix mit der Übergangs-oder Lanthanoidverbindung in Kontakt gebracht wird.

Vorzugsweise erfolgt dieses Inkontaktbringen durch Eintauchen, Besprühen oder Imprägnieren der Ausgangsmatrix mit einer Lösung der Übergangs- oder Lanthanoidverbindung in einem geeigneten Lösungsmittel, besonders bevorzugt Wasser oder Gemische aus Wasser und organischen Lösungsmitteln. Die Konzentration der Übergangsmetall- oder Lanthanoidverbindung bezogen auf die Polysaccharid-Ausgangsmatrix beträgt 0,2 bis 500 mmol/kg Ausgangsmatrix, bevorzugt 1 bis 200 mmol/kg Ausgangsmatrix und am meisten bevorzugt 2 bis 100 mmol/kg Ausgangsmatrix.

Das nachfolgende Pfropfen in Schritt B) erfolgt bevorzugt mit einer Pfropfflotte, umfassend die organische Säure, die mindestens eine ethylenische Monomerverbindung und Wasser oder mindestens ein organisches Lösungsmittel bzw. Gemische davon. Die Pfropfflotte kann als Lösung oder Emulsion vorliegen. Die Konzentration der organischen Säure in der Pfropfflotte beträgt 0,01 bis 100 mmol/l, bevorzugt 0,05 bis 30 mmol/l und besonders bevorzugt 0,1 bis 3 mmol/l.

Die Konzentration der mindestens einen ethylenischen Monomerverbindung in der Pfropfflotte beträgt unter 50 Gew%, bevorzugt unter 20 Gew% und am meisten bevorzugt unter 5 Gew%.

Bei den beiden vorgenannten Verfahren zur Herstellung der erfindungsgemäßen Polysaccharidmatrix können nach dem Schritt B) des Pfropfens die funktionellen Gruppen des auf die Polysaccharid-Ausgangsmatrix gepfropften Polymers in einem anschließenden Schritt C) mit mindestens einem Liganden, der zur Wechselwirkung mit in Fluiden enthaltenen Adsorbenden befähigt ist, umgesetzt werden. Dieser Ligand weist erfindungsgemäß, wie vorstehend beschrieben, kationische und/oder anionische Gruppen auf und kann eine "polymeranaloge Reaktion" mit den funktionellen Gruppen der Monomereinheiten des in Schritt B) aufgepfropften Polymers eingehen, so daß diese Monomereinheiten in durch den mindestens einen Liganden funktionalisierte Wiederholungseinheiten des aufgepfropften Polymers überführt werden. Alternativ können zwei verschiedene Liganden eingesetzt werden, von denen der erste Ligand eine anionische und der zweite Ligand eine hydrophobe Gruppe aufweist.

Bei beiden erfindungsgemäßen Verfahren ist die Polysaccharid-Ausgangsmatrix bevorzugt ausgewählt aus der Gruppe, bestehend aus Celluloseestern, Cellulosehydrat, Celluloseethern, Agarose, Chitin, Chitosan, Dextran oder Kombinationen davon.

Weitere Varianten der beiden erfindungsgemäßen Verfahren zur Herstellung der erfindungsgemäßen Polysaccharidmatrix, bei welchen Celluloseester als Ausgangsmatrizen eingesetzt werden, umfassen einen zusätzlichen Schritt der Behandlung der Polysaccharidmatrix mit einem basischen Medium, um Estergruppen der Polysaccharidmatrix zu hydrolysieren.
Bei einer Variante, bei welcher nach dem Schritt des Pfropfens B) in einem weiteren Schritt C) mindestens ein Ligand mit funktionellen Gruppen des Polymers reagiert, erfolgt die Behandlung der Polysaccharidmatrix mit dem basischen Medium während oder nach Schritt C).
Bei einer weiteren Variante, bei welcher auf den Schritt C) des Umsetzens mit einem Liganden verzichtet wird, erfolgt die Behandlung der nassen oder getrockneten Polysaccharidmatrix mit einem basischen Medium nach dem Schritt B) des Pfropfens. Die Behandlung mit einem basischen Medium wird vorzugsweise mit einem wässrigen Medium durchgeführt, welches eine alkalische Verbindung, besonders bevorzugt ein Alkalimetallhydroxid, aufweist. Besonders bevorzugt sind wäßrige Lösungen von Natrium- oder Kaliumhydroxid. Es können auch Gemische aus einem Alkalimetallhydroxid und anderen alkalischen Verbindungen, wie Alkalimetallcarbonaten (z. B. Natriumcarbonat, Kaliumcarbonat oder Cäsiumcarbonat), tri-Natriumphosphat und/oder tri-Kaliumphosphat eingesetzt werden.

Die Bedingungen für die basische Behandlung der Polysaccharidmatrix sind vorzugsweise so ausgewählt, dass etwaige Epoxidgruppen des aufgepfropften Polymers nicht oder nur in geringem Maß hydrolysieren. Die Konzentration der alkalischen Verbindung in dem basischen Medium kann in weiten Grenzen variiert werden und wird so optimiert, daß der Erhalt von Epoxidgruppen, die Verseifungsreaktion der Estergruppen der Matrix oder die polymeranaloge Reaktion bevorzugt abläuft. Gemäß einer besonders bevorzugten Ausführungsform der vorliegenden Erfindung wird ein basisches Medium aus Wasser und Natriumhydroxid verwendet, wobei die Konzentration des Natriumhydroxids in der Lösung in einem Bereich von 0,1 bis 10 Gew.-%, besonders bevorzugt in einem Bereich von 0,4 bis 4 Gew.-% liegt.
Das basische Medium kann ein oder mehrere Additiv(e) enthalten. Geeignete Additive sind vor allem Salze, wie Natriumchlorid, Natriumsulfat und Natriumacetat oder organische Lösungsmittel. Das organische Lösungsmittel wird vorzugsweise aus der Gruppe von Alkoholen, Ketonen oder Ethern ausgewählt. Besonders bevorzugt ist Ethanol, Methanol, Ethylenglycol, Propylenglycol, Glycerin, Aceton, Dioxan, N-Methylpyrrolidon, Diglyme oder Gemische davon.

Die Verwendung der erfindungsgemäßen Polysaccharidmatrizen erfolgt in der Stofftrennung zur Entfernung partikulärer, kolloidaler und gelöster Komponenten aus flüssigen Medien. Die abzutrennenden Komponenten sind hierbei bevorzugt unerwünschte biologische Kontaminanten. Besonders bevorzugt umfassen die abzutrennenden Komponenten Kontaminanten aus Zellkulturlösungen, wie z. B. Zellen, Zellbruchstücke, Zellproteine, DNS, Viren, Endotoxine. Der Abtrennung umfasst hierbei mindestens eine der unerwünschten biologischen Kontaminanten aus dem flüssigen Medium, während Zielprodukte, die ebenfalls in dem flüssigen Medium enthalten sind, aufgereinigt als Eluat isoliert werden. Unter Zielprodukten im Sinne der vorliegenden Erfindung werden Biomoleküle verstanden. Besonders bevorzugt werden unter Zielprodukten Proteine, Oligopeptide, Polypeptide oder Hormone verstanden. Unter flüssigen Medien werden dabei alle Medien verstanden, die ein Inkontaktbringen der abzutrennenden Bestandteile mit der erfindungsgemäßen Polysaccharidmatrix ermöglichen.
Ein Vorteil der vorliegenden Erfindung besteht darin, daß durch geeignete Auswahl der mindestens einen ethylenischen Monomerverbindung und des mindestens einen Liganden Polysaccharidmatrizen mit hoher Proteinbindungskapazität für die jeweils gewünschte Stofftrennungsapplikation bereitgestellt werden können.

### Beispiele

Die vorliegende Erfindung wird im Folgenden durch die Beispiele näher erläutert, ohne jedoch durch diese in irgendeiner Weise eingeschränkt zu sein.

Sämtliche Erwähnungen einer CA-Membran in den Beispielen beziehen sich auf einen mit Polyester-Vlies verstärkten Typ einer Celluloseacetat-Membran mit einem Porendurchmesser von ca. 3 µm (gemessen mit einem Coulter-Porometer Typ "Capillary Flow Porometer 6.0", CAPWIN Software System, Fa. Porous Materials Inc.), welche einen Wasserdurchfluss von 600-700 ml/(min _{*} bar _{*} cm²) aufweist. Alle Durchflussangaben für die erfindungsgemäßen Membranen und die Vergleichsmembran sind in ml/(min _{*} bar _{*} cm²) für eine 20 mM-Tris(hydroxymethyl)aminomethan-(TRIS)/HCl-Pufferlösung bei pH = 7,3 angegeben. Alle statischen Bindungskapazitätsangaben sind in mg/cm² angegeben.
Die dynamische Bindungskapazität in mg/ml wird als die Menge Absorbend definiert, die (als Lösung) bis zu dem Zeitpunkt durch die erfindungsgemäße Polysaccharidmatrix gelaufen ist, an dem die Konzentration des Adsorbenden im Ablauf der Polysaccharidmatrix einen definierten Bruchteil der Konzentration des Adsorbenden im Zulauf aufweist. Aus praktischen Gründen werden 10 % der Zulaufkonzentration angegeben.
Sofern nichts anderes angegeben ist, beziehen sich Prozentangaben auf das Gewicht.

### Beispiel 1: Pfropfung von Glycidylmethacrylat auf eine mit einer organischen Säure behandelte CA-Membran, Variante ohne Vernetzer, Lanthanoidverbindung in der Pfropfflotte

8 CA-Membranronden mit einer Gesamtfläche von 240 cm² wurden mit Reverse-Osmose-(RO)-Wasser benetzt, 10 min mit RO-Wasser gespült und anschließend in 1000 g einer 0,1-mmol Lösung einer in der Tabelle 1 genannten organischen Säure in Wasser 10 min eingetaucht. Danach wurden die Membranen 15 min bei 80 °C in einem Umlufttrockenschrank getrocknet. 240 cm² der mit der organischen Säure behandelten CA-Membranen wurden im Gasraum eines auf 22 °C temperierten Reaktionsgefäßes befestigt. 14,6 g Glycidylmethacrylat und 0,45 g Arlatone^{®} G (Fa. Atlas) wurden in dem Reaktionsgefäß vorgelegt und zusammen mit den Membranen 30 min mit Stickstoff begast. Eine Lösung von 1,5 g Ce(IV)-sulfat, 5,0g 20%-iger Schwefelsäure und 478 g RO-Wasser wurde 30 min mit Stickstoff begast und in das Reaktionsgefäß unter Stickstoff überführt. Das Gemisch wurde 5 min gerührt. Danach wurden die Membranen aus dem Gasraum des Reaktionsgefäßes in die Lösung eingetaucht und nach ca. 5 s wieder aus der Lösung in den Gasraum überführt. Nach weiteren 20 min wurden die Membranen zunächst 5 min mit 0,2%iger wäßriger Schwefelsäure und dann mit fließendem RO-Wasser 10 min gespült. Danach wurden die Membranen 30 min in 0,5 M NaOH geschüttelt und 10 min mit fließendem RO-Wasser gespült.

### Vergleichsbeispiel 1:

Die Vergleichsmembran der Tabelle 1 wurde nach Beispiel 1 hergestellt, wobei vor Durchführen des Pfropfens keine Behandlung der CA-Membranen mit einer der organischen Säuren aus Tabelle 1 erfolgte.

Die Daten zum Durchfluß und zur Kapazität für Rinderserumalbumin der nach Beispiel 1 gepfropften und nach Beispiel 5 mit quarternären Ammoniumgruppen umgesetzten Membranen im Vergleich zu CA-Membranronden ("Vergleichsmembran"), die nicht mit einer organischen Säure behandelt wurden, aber ansonsten analog erst nach Beispiel 1 und dann nach Beispiel 5 umgesetzt wurden, sind in Tabelle 1 enthalten. Durch den Einsatz von mit organischer Säure behandelten Membranen kann die statische Bindungskapazität für RSA um das 1,5- bis 8,4-fache im Vergleich zu der Vergleichsmembran erhöht werden, wenn Citronensäure, Äpfelsäure, Weinsäure, Fumarsäure, Maleinsäure, Bernsteinsäure, Oxalsäure, Malonsäure, Ascorbinsäure, D-Glucuronsäure oder Milchsäure als organische Säure eingesetzt werden.

**Tabelle 1:**

| Organische Säure | Durchfluss [ml/(cm²*min*bar)] | Kapazität Rinderserumalbumin (RSA) [mg/cm²] |
|---|---|---|
| Citronensäure | 51 | 2,85 |
| D-(+)-Äpfelsäure | 70 | 2,29 |
| L-(-)-Äpfelsäure | 51 | 2,22 |
| L-(+)-Weinsäure | 97 | 1,87 |
| Tricarballylsäure | 401 | 0,30 |
| Fumarsäure | 242 | 0,70 |
| Maleinsäure | 286 | 0,52 |
| Bernsteinsäure | 257 | 0,75 |
| trans-Aconitsäure | 403 | 0,30 |
| cis-Aconitsäure | 404 | 0,34 |
| Oxalsäure | 151 | 1,22 |
| Malonsäure | 179 | 1,02 |
| L-(+)-Ascorbinsäure | 121 | 1,23 |
| D-Glucuronsäure | 388 | 0,52 |
| Glycolsäure | 443 | 0,44 |
| DL-Milchsäure | 272 | 1,03 |
| 3-Hydroxy-2-butanone | 483 | 0,34 |
| 4-Hydroxy-3-hexanone | 459 | 0,32 |
| Vergleichsmembran | 368 | 0,34 |

### Beispiel 2: Pfropfung von Glycidylmethacrylat auf eine mit einer organischen Säure behandelte CA-Membran, Variante mit Vernetzer, Lanthanoidverbindung in der Pfropfflotte

8 CA-Membranronden mit einer Gesamtfläche von 240 cm² wurden mit RO-Wasser benetzt, 10 min mit RO-Wasser gespült und anschließend in 1000 g einer 0,1 mM Lösung von Citronensäure in Wasser 10 min eingetaucht. Danach wurden die Membranen 15 min bei 80 °C in einem Umlufttrockenschrank getrocknet. 240 cm² CA-Membranen wurden im Gasraum eines auf 22 °C temperierten Reaktionsgefäßes befestigt. 14,5 g Glycidylmethacrylat, 0,07 g Ethylenglycoldimethacrylat und 0,45g Arlatone^{®} G (Fa. Atlas) wurden in dem Reaktionsgefäß vorgelegt und zusammen mit den Membranen 30 min mit Stickstoff begast. Eine Lösung von 1,5 g Ce(IV)-sulfat, 5,0 g 20%-iger Schwefelsäure und 478 g RO-Wasser wurde 30 min mit Stickstoff begast und in das Reaktionsgefäß unter Stickstoff überführt. Das Gemisch wurde 5 min gerührt. Danach wurden die Membranen aus dem Gasraum des Reaktionsgefäßes in die Lösung eingetaucht und nach ca. 5 s wieder aus der Lösung in den Gasraum überführt. Nach weiteren 20 min wurden die Membranen zunächst 5 min mit 0,2%iger wäßriger Schwefelsäure und dann mit fließendem RO-Wasser 10 min gespült. Anschließend wurden die Membranen 30 min in 0,5 M NaOH geschüttelt und 10 min mit fließendem RO-Wasser gespült.

### Beispiel 3: Pfropfung von Glycidylmethacrylat auf eine mit einer Lanthanoidverbindung behandelte CA-Membran, Variante mit Vernetzer, organische Säure in der Pfropfflotte

8 CA-Membranronden mit einer Gesamtfläche von 240 cm² wurden mit RO-Wasser benetzt, 10 min mit RO-Wasser gespült und 5 min in 500 g einer Lösung aus 1,5 g Cer-(IV)-sulfat, 5,0 g 20%-iger Schwefelsäure und 478 g RO-Wasser eingetaucht. Danach wurden die Membranen 15 min bei 60 °C in einem Umlufttrockenschrank getrocknet. 240 cm² CA-Membran wurden im Gasraum eines auf 22 °C temperierten Reaktionsgefäßes befestigt. Eine Lösung aus 10,0 g Glycidylmethacrylat, 0,05 g Ethylenglycoldimethacrylat, 0,05 g Citronensäure und 490 g RO-Wasser wurde, zusammen mit den Membranen im Gasraum des Reaktionsgefässes, 30 min mit Stickstoff begast. Danach wurden die Membranen aus dem Gasraum des Reaktionsgefässes in die Lösung eingetaucht und nach ca. 5 s wieder aus der Lösung in den Gasraum überführt. Nach weiteren 20 min wurden die Membranen für 10 min in 500 g 0,2%-iger Schwefelsäure geschüttelt. Nachfolgend wurden die Membranen 10 min mit fliessendem RO-Wasser gespült. Danach wurden die Membranen 30 min in 0,5 M NaOH geschüttelt und 10 min mit fließendem RO-Wasser gespült.

### Beispiel 4: Pfropfung von zwei verschiedenen Monomeren (Glycidylmethacrylat und Benzylmethacrylat) auf eine mit einer Säure behandelte CA-Membran, Variante mit Vernetzer, Lanthanoidverbindung in der Pfropfflotte

8 CA-Membranronden mit einer Gesamtfläche von 240 cm² wurden mit RO-Wasser benetzt, 10 min mit RO-Wasser gespült und anschließend in 1000 g einer 0,1 mM Lösung von Citronensäure in Wasser 10 min eingetaucht. Danach wurden die Membranen 15 min bei 80 °C in einem Umlufttrockenschrank getrocknet. 240 cm² CA-Membranen wurden im Gasraum eines auf 22 °C temperierten Reaktionsgefäßes befestigt. 13,5 g Glycidylmethacrylat, 0,07 g Ethylenglycoldimethacrylat, 1,0 g Benzylmethacrylat und 0,45 g Arlatone^{®} G (Fa. Atlas) wurden in dem Reaktionsgefäß vorgelegt und zusammen mit der Membran 30 min mit Stickstoff begast. Eine Lösung von 1,5 g Ce(IV)-sulfat, 5,0 g 20%-iger H₂SO₄ und 478 g RO-Wasser wurde 30 min mit Stickstoff begast und in das Reaktionsgefäß unter Stickstoff überführt. Das Gemisch wurde 5 min gerührt. Danach wurden die Membranen aus dem Gasraum des Reaktionsgefäßes in die Lösung eingetaucht und nach ca. 5 s wieder aus der Lösung in den Gasraum überführt. Nach weiteren 20 min wurden die Membranen mit fließendem RO-Wasser 10 min gespült. Danach wurden die Membranen 30 min in 0,5 M NaOH geschüttelt und 10 min mit fließendem RO-Wasser gespült.

### Beispiel 5: Polymeranaloge Reaktion zur Einführung eines Liganden (Quaternäre Ammoniumgruppe)

Die gepfropften Membranen aus den Beispielen 1, 2 bzw. 3 wurden sukzessiv 35 Minuten bei 30 °C mit einer 10%igen wäßrigen Lösung von Trimethylamin und 5 Minuten bei Raumtemperatur mit 5%iger wäßriger Schwefelsäure-Lösung behandelt und danach 10 Minuten mit fließendem Wasser gespült.

### Beispiel 6: Polymeranaloge Reaktion zur Einführung eines Liganden (Sulfonsäuregruppe)

Die Membranen aus Beispiel 1 wurden in einer Lösung von 380 g Na₂SO₃, 40,0 g Na₂HPO_{4*}H₂O, 100 g Tetrabutylammoniumbisulfat und 1200 g Wasser mit 32-%iger wäßriger Natronlauge auf einen pH-Wert von 8,0 eingestellt, 45 min bei 80 °C behandelt und danach 10 Minuten mit fließendem Wasser, 5 Minuten mit 35 g 1%iger wäßriger Salzsäure, 2 x 5 min mit je 30 g wässriger 1 M NaCl-Lösung, 5 min mit 500 g 5%iger wäßriger Schwefelsäure und 10 Minuten mit fließendem Wasser gespült.

### Beispiel 7: Polymeranaloge Reaktion zur Einführung von zwei verschiedenen Liganden (Sulfonsäuregruppe und Phenylamino-Gruppe)

312 g einer wässrigen Lösung aus 18 Gew.-% Na₂SO₃, 1 Gew.-% Anilin, 5 Gew.-% Tetrabutylammoniumbisulfat, 2 Gew.-% Na₂HPO_{3*}2H₂O (mit 32%-iger NaOH auf pH 8 eingestellt) wurden auf 35 °C erhitzt. Die Membranen aus Beispiel 1 wurden zugegeben und die Reaktionslösung wurde 16 h bei 35 °C gerührt. Die Membranen wurden dann 10 Minuten mit fließendem RO-Wasser, 15 Minuten mit 200 g 5%iger wäßriger Schwefelsäure, 2 x 10 Minuten mit 200 g 50 mM wäßriger Na₂SO₄-Lösung, 5 Minuten mit fließendem RO-Wasser, 2 x 10 Minuten mit 300 g Ethanol und abschließend 5 Minuten mit fließendem RO-Wasser gespült.

### Beispiel 8: Polymeranaloge Reaktion zur Einführung eines Liganden (Sulfonsäuregruppe)

Die Membranen aus Beispiel 2 wurden wie im Beispiel 6 beschrieben umgesetzt.

### Vergleichsbeispiel 2:

Polymeranaloge Reaktion zur Einführung eines Liganden (Sulfanilsäuregruppe)

Eine 2%-ige Lösung von Sulfanilsäure (4-Aminobenzolsulfonsäure) in RO-Wasser wurde mit 32%-iger NaOH auf einen pH-Wert von 11,3 eingestellt. Die Membranen aus Beispiel 2 wurden zugegeben und die Reaktionslösung wurde 3 h bei 65 °C gerührt. Die Membranen wurden 10 Minuten mit fließendem RO-Wasser, 15 Minuten mit 200 g 5%-iger wäßriger Schwefelsäure, 2 x 10 Minuten mit 200 g 50 mM wäßriger Na₂SO₄-Lösung, 5 Minuten mit fließendem RO-Wasser, 2 x 10 Minuten mit 300 g Ethanol und abschließend 25 Minuten mit fließendem RO-Wasser gespült.

### Beispiel 9: Polymeranaloge Reaktion zur Einführung von zwei verschiedenen Liganden (Sulfonsäuregruppe und Sulfanilsäure-Gruppe)

Die im Beispiel 6 beschriebene Sulfonierungslösung wurde mit RO-Wasser auf 2/3 der Ausgangskonzentration der gelösten Bestandteile verdünnt und dann mit Sulfanilsäure (Aldrich S5263) versetzt, bis der Sulfanilsäure-Gehalt in der verdünnten Sulfonierungslösung 2 Gew.-%- bzw. 4 Gew.-% betrug. Nach Lösen der Sulfanilsäure wurde der pH-Wert mit 32%-iger NaOH auf 11,3 eingestellt. Die Membranen aus Beispiel 2 wurden zugegeben und die Reaktionslösung wurde 3 h bei 65 °C gerührt. Die Membranen wurden 10 Minuten mit fließendem RO-Wasser, 15 Minuten mit 200 g 5%-iger wäßriger Schwefelsäure, 2 x 10 Minuten mit 200 g 50 mM wäßriger Na₂SO₄-Lösung, 5 Minuten mit fließendem RO-Wasser, 2 x 10 Minuten mit 300 g Ethanol und abschließend 25 Minuten mit fließendem RO-Wasser gespült.

### Beispiel 10: Polymeranaloge Reaktion zur Einführung von quaternären Ammoniumgruppen unter Vernetzung des aufgepfropften Polymers

Die Membranen gemäß des Beispiels 1 wurden 35 Minuten bei 30 °C in einer wässrigen Lösung von 10 Gew.- % Trimethylamin und 2,5 Gew.-% Diazabicyclooctan und 5 Minuten bei Raumtemperatur in 5%iger Schwefelsäure behandelt und danach 10 Minuten mit fließendem Wasser gespült.

### Beispiel 11: Polymeranaloge Reaktion zur Einführung eines Liganden mit sekundärer und tertiärer Aminogruppe

Die Membranen aus Beispiel 1 wurden 1 h bei 60 °C in einer 20 %igen, wässrigen Lösung von N-Diethyl-N'-dimethyl-l,3-propandiamin und 5 Minuten bei Raumtemperatur in 5%iger Schwefelsäure behandelt und danach 10 Minuten mit fließendem Wasser gespült.

### Beispiel 12: Polymeranaloge Reaktion zur Einführung eines Liganden mit tertiärer Aminogruppe

Die Membranen aus Beispiel 2 wurden sukzessiv 1 h bei 60 °C in einer 1%igen, wässrigen Lösung von N,N-Dimethylbenzylamin und 5 Minuten bei Raumtemperatur in 5%iger Schwefelsäure behandelt und danach 10 Minuten mit fließendem Wasser gespült.

### Auswertung der Membranen:

Die Auswertung der erhaltenen Membranen erfolgte in der nachstehend beschriebenen Weise:

### 1) Durchflussbestimmung

Membranen mit einer aktiven Membranfläche von 12,5 cm² wurden jeweils in ein Gehäuse eingebaut und es wurde die Zeit für die Filtration von 100 ml Puffer (Gemisch aus 10 mM KPi-Puffer (0,01 Mol/Liter-Kaliumphosphatpuffer mit pH = 7,0, welcher durch Mischen von KH₂PO₄-Lösung mit K₂HPO₄-Lösung hergestellt wurde) und 1 M wäßriger NaCl-Lösung, auf einen pH-Wert von 7,0 eingestellt) gemessen. Die in der Tabelle 2 wiedergegebenen Durchflussangaben für die mit Liganden umgesetzten Membranen beziehen sich auf den entsprechenden TRIS/HCl-Puffer bei pH = 7,3. Es wurden die gleichen Puffer verwendet wie für die unten beschriebenen Bestimmungen der Bindungskapazitäten.

### 2) Bestimmung der statischen Bindungskapazität von positiv geladenen Membranen mit Aminliganden

Membranen mit einer aktiven Membranfläche von jeweils 17,6 cm² wurden in 35 ml 20 mM TRIS/HCl (pH 7,3) 3 x 5 min mit etwa 80 Umdrehungen pro Minute (Upm) geschüttelt. Danach wurden die Membranen in 35 ml einer Lösung von 2 mg/ml Rinderserumalbumin (RSA)-Lösung (Fa. Kräber, Ellerbeck, Best.-Nr. 041 80 10 900, Chargen-Nr. 49 09 20 52) in 20 mM TRIS/HCl (pH = 7,3) 12-18 Stunden bei 20-25 °C mit etwa 80 Upm geschüttelt. Anschließend wurden die Membranen 2 x 15 Minuten mit jeweils 35 ml 20mM TRIS/HCl (pH = 7,3) gespült. Danach wurden die Membranproben in 20 ml eines Gemisches aus einer Lösung von 20 mM TRIS/HCl und 1 M wäßriger Natriumchloridlösung bei pH = 7,3 geschüttelt. Die Menge des eluierten Rinderserumalbumins wurde durch Messung der optischen Dichte (OD) bei 280 nm bestimmt.

### 3) Bestimmung der statischen Bindungskapazität von negativ geladenen Membranen mit Sulfonsäure- und/oder Sulfanilsäure-Ligand gemäß der Beispiele 6, 7, 8 und 9 sowie des Vergleichsbeispiels 2

Membranen mit einer aktiven Membranfläche von jeweils 17,6 cm² wurden in 35 ml 10 mM KPi-Puffer (pH 7,0) 3mal 5 Minuten mit etwa 80 Upm geschüttelt. Danach wurden die Membranen in 35 ml einer Lösung von 2 mg/ml Lysozym (Sigma Aldrich Chemie, Taufkirchen, Bestell-Nr. L6876, Lot-Nr. 114K0626) in 10 mM KPi-Puffer (pH 7,6) 12-18 Stunden bei 20 - 25 °C mit etwa 80 Upm geschüttelt. Anschließend wurden die Membranen 2 x 15 Minuten mit jeweils 35 ml 10 mM KPi-Puffer (pH 7,0) gespült. Danach wurden die Membranen in 20 ml eines Gemisches aus 10 mM KPi-Puffer und wäßriger 1 M Natriumchloridlösung (pH = 7,3) geschüttelt. Die Menge des eluierten Lysozyms wurde durch Messung der optischen Dichte (OD) bei 280 nm bestimmt.

### 4) Bestimmung der statischen Bindungskapazitäten von negativ geladenen Membranen nach Beispiel 8 und 9 sowie nach Vergleichsbeispiel 2 bei verschiedenen Konzentrationen von 150 mM bzw. 300 mM NaCl

Die statischen Bindungskapazitäten für Membranen nach dem Vergleichsbeispiel 2 sowie nach den Beispielen 8 und 9 wurden nach dem folgenden Verfahren bestimmt: Membranen mit einer aktiven Membranfläche von jeweils 17,6 cm² wurden in 35 ml 20 mM Acetat-Puffer (pH = 5,0, wahlweise auf eine NaCl-Konzentration von 150 mM oder 300 mM eingestellt) 3mal 5 Minuten mit etwa 80 Umdrehungen/min geschüttelt. Danach wurden die Membranen in 35 ml einer Lösung von 1 mg/ml Globulin G-5009 Sigma (Sigma Aldrich Chemie, Quelle: Rinderblut) in 20 mM Acetat-Puffer (pH = 5,0, wahlweise auf eine NaCl-Konzentration von 150 mM oder 300 mM eingestellt) 12-18 Stunden bei 20 - 25 °C mit etwa 80 Umdrehungen/min geschüttelt. Anschließend wurden die Membranen 2 x 15 Minuten mit jeweils 35 ml 20 mM Acetat-Puffer (pH = 5,0, wahlweise auf eine NaCl-Konzentration von 150 mM oder 300 mM eingestellt) behandelt. Danach wurden die Membranen jeweils in 20 ml eines 20 mM Acetat-Puffers (pH = 5,0 + 1 M NaCl) geschüttelt. Die Menge des eluierten Globulins wurde durch Messung der optischen Dichte (OD) bei 280 nm bestimmt.

### 5) Bestimmung der dynamischen Bindungskapazität von positiv geladenen Membranen mit Aminliganden

3 Lagen Membran wurden in einen Membranhalter eingespannt. Der Membranstapel wies eine Membranfläche von 15 cm² eine Anströmfläche von 5 cm² und eine Betthöhe (Dicke des Membranstapels) von 750 µm in dem Membranhalter auf. Die Membranen in dem Membranhalter wurden mit 20 mM TRIS/HCI-Puffer (pH =7,3) geflutet, um die Luft zu verdrängen und dann an eine FPLC-Anlage "Äkta^{®} Explorer 100" der Firma General Electric Health Care angeschlossen.

Danach wurden die Membranen bzw. der Membranstapel mit einem zwei Schritte umfassenden Testprogramm hinsichtlich der RSA(Rinderserumalbumin)-Bindung untersucht. Die zwei Schritte des Testprogramms sind nachfolgend angegeben:
1. Äquilibrieren der Membranen mit 10 ml eines 50 mM 20 mM TRIS/HCl-Puffers bei pH =7,3
2. Beladen der Membran mit 1 mg/ml RSA in 20 mM TRIS/HCl-Puffer bei pH =7,3, bis die Konzentration im UV-Detektor 10 % der Ausgangskonzentration beträgt.

Beide Schritte wurden bei einer Flußgeschwindigkeit von 10 ml/min durchgeführt. Bei allen Schritten wurde die Absorbanz bei 280 nm im UV-Detektor hinter der Membraneinheit gemessen. Die Fläche oberhalb der so aufgezeichneten Durchbruchskurve wurde nach dem Abziehen des Totvolumens integriert und daraus die dynamische Bindungskapazität bei 10 % Durchbruch berechnet.

Die nachfolgende Tabelle 2 enthält Daten zu erfindungsgemäßen Membranen, bei welchen die funktionellen Gruppen des aufgepfropften Polymers mit Liganden umgesetzt sind. Die Angabe "Beispiel 1, 5" bedeutet beispielsweise, daß Ausgangsmembranen zunächst gemäß Beispiel 1 umgesetzt wurden und nachfolgend gemäß Beispiel 5 mit einem Liganden weiterumgesetzt wurden.

**Tabelle 2A:**

| Membran Polymeranaloge Reaktion | Dicke [µm] | Durchfluss | |
|---|---|---|---|
| | | [ml/(min∗bar∗cm²)] | [cm/h]* |
| 1) Beispiel 1, 5 | 205 | 51 | 63 |
| 2) Beispiel 1, 6 | 210 | 87 | 110 |
| 3) Beispiel 1, 7 | 205 | 138 | 170 |
| 4) Beispiel 1, 10 | 205 | 112 | 138 |
| 5) Beispiel 1, 11 | 205 | 65 | 80 |
| 6) Beispiel 2, 5 | 210 | 104 | 131 |
| 7) Beispiel 2, 12 | 205 | 50 | 62 |
| 8) Beispiel 3, 5 | 210 | 115 | 145 |
| 9) Beispiel 4, 5 | 215 | 81 | 104 |

**Tabelle 2B:**

| Membran Polymeranaloge Reaktion | Kapazität | | 10%DBK** | |
|---|---|---|---|---|
| | [mg/cm²] | [mg/ml]⁺ | [mg/cm²] | [mg/ml]⁺ |
| 1) Beispiel 1, 5 | 2,85 | 139 | 2,15 | 105 |
| 2) Beispiel 1, 6 | 1,77 | 84 | - | - |
| 3) Beispiel 1, 7 | 2,62 | 128 | - | - |
| 4) Beispiel 1, 10 | 2,22 | 108 | - | - |
| 5) Beispiel 1, 11 | 2,28 | 111 | 1,73 | 84 |
| 6) Beispiel 2, 5 | 2,35 | 112 | - | - |
| 7) Beispiel 2, 12 | 2,64 | 129 | - | - |
| 8) Beispiel 3, 5 | 2,05 | 98 | - | - |
| 9) Beispiel 4, 5 | 2,03 | 94 | 1,68 | 78 |

| | | | | |
|---|---|---|---|---|
| * Durchfluss durch einen Membranstapel mit 1cm² Anströmfläche und 1 cm Höhe bei 1 bar gemessen mit 20 mM TRIS/HCl-Puffer bei pH = 7,3 ** 10 % DBK = dynamische Bindungskapazität bei 10 % Durchbruch ⁺ Umrechnung der Kapazität bezogen auf die Membranfläche in cm² in die Kapazität bezogen auf das Membranvolumen in ml unter Berücksichtigung der Membrandicke | | | | |

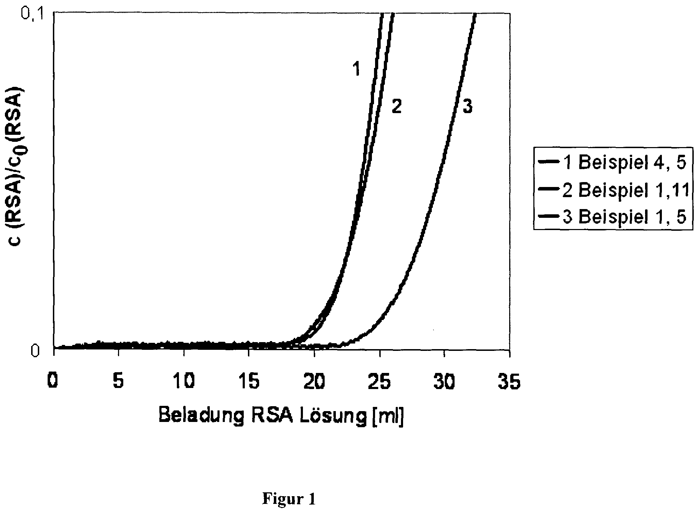

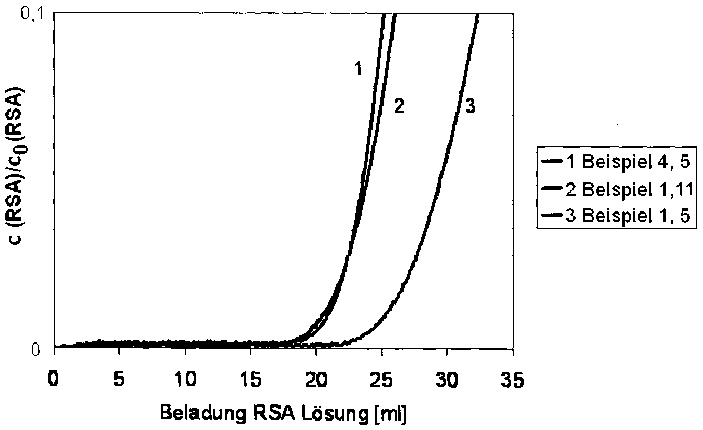

Figur 1 zeigt Durchbruchskurven für Rinderserumalbumin für Membranen, die nach den Einträgen 1), 5) und 9) der Tabelle 2B) hergestellt worden sind. Die y-Achse zeigt das Verhältnis aus der Konzentration c(RSA) im Ablauf und der Konzentration c₀(RSA) im Zulauf, während die x-Achse die Beladung mit RSA angibt.

Die nachfolgende Tabelle 3 enthält Daten der Membran nach dem Vergleichsbeispiel 2 sowie der erfindungsgemäßen Membranen nach den Beispielen 8 und 9, welche mindestens einen Liganden mit einer anionischen Gruppe aufweisen. Die Membranen des Vergleichsbeispiels 2, welche nur den Sulfanilsäureliganden aufweisen, zeigen nur sehr geringe statische Bindungskapazitäten für Globulin und für Lysozym, während Membranen, welche sowohl Sulfonsäure- als auch Sulfanilsäuregruppen als Liganden aufweisen, auch bei hohen Salzkonzentrationen eine hohe statische Bindungskapazität für Lysozym und Globulin zeigen.

**Tabelle 3A:**

| | Durchfluss* [ml/(min bar cm²)] | Globulin-Kapazität [mg/cm²] | |
|---|---|---|---|
| | | 150 mM NaCl | 300 mM NaCl |
| Beispiel 8 | 50 | 1,17 | 0,09 |
| Vergleichsbeispiel 2 | 330 | 0,05 | 0,02 |
| Beispiel 9 | | | |
| c₀**=2Gew.-% Sulfanilsäure | 50 | 2,13 | 0,43 |
| Beispiel 9 | | | |
| c₀** = 4 Gew.-% Sulfanilsäure | 40 | 2,40 | 0,90 |

**Tabelle 3B:**

| | Dicke | Lysozym-Kapazität |
|---|---|---|
| | [µm] | [mg/cm²] |
| Beispiel 8 | 210 | 2,32 |
| Vergleichsbeispiel 2 | 210 | 0,07 |
| Beispiel 9 | | |
| c₀**=2Gew.-% Sulfanilsäure | 215 | 2,51 |
| Beispiel 9 | | |
| c₀** = 4 Gew.-% Sulfanilsäure | 210 | 2,66 |

| | | |
|---|---|---|
| * Durchfluss gemessen mit 20 mM Acetat-Puffer pH = 5,0 + 150mM NaCl ** Konzentration an Sulfanilsäure in Gew.-% in der Sulfonierungslösung nach Beispiel 9 | | |

## Patentansprüche

1. Poröse, nicht partikuläre, konvektiv permeable Polysaccharidmatrix, auf deren Oberfläche ein aufgepfropftes Polymer aus mindestens einer ethylenischen Monomerverbindung fixiert ist, hergestellt durch Pfropfen
- einer porösen, nicht partikulären, konvektiv permeablen Polysaccharid-Ausgangsmatrix mit
- der mindestens einen ethylenischen Monomerverbindung mit Epoxid-Gruppen in Anwesenheit
- einer organischen Säure, wobei die organische Säure ausgewählt ist aus der Gruppe bestehend aus Citronensäure, Äpfelsäure, Weinsäure, Fumarsäure, Maleinsäure, Bernsteinsäure, Oxalsäure, Malonsäure, Ascorbinsäure, Glucuronsäure, Milchsäure oder Kombinationen davon, und
- einer Übergangsmetall- oder Lanthanoidverbindung, ausgewählt aus der Gruppe der Cer-, Mangan-, Eisen-, Nickel-, Cobalt-, Vanadiumverbindungen oder Kombinationen davon, zur Erzeugung eines auf die Polysaccharid-Ausgangsmatrix gepfropften Polymers mit Epoxid-Gruppen, wobei nach dem Pfropfen die Epoxid-Gruppen des auf die Polysaccharid-Ausgangsmatrix gepfropften Polymers mit mindestens einem Liganden, der zur Wechselwirkung mit in Fluiden enthaltenen Adsorbenden befähigt ist und kationische und/oder anionische Gruppen aufweist, umgesetzt werden.

2. Polysaccharidmatrix nach Anspruch 1, wobei die Polysaccharid-Ausgangsmatrix aus Polysaccharid besteht.

3. Polysaccharidmatrix nach Anspruch 1, wobei die Polysaccharid-Ausgangsmatrix ein poröser Träger mit einer Polysaccharidbeschichtung ist.

4. Polysaccharidmatrix nach Anspruch 3, wobei der poröse Träger aus einem Polymer besteht, welches ausgewählt ist aus der Gruppe, bestehend aus Polyamid, Poly(ether)sulfon, Polyvinylidendifluorid, Polyacrylnitril, Polyvinylchlorid, Polypropen, Polyethen, Polytetrafluorethen, deren Copolymeren oder Gemischen davon.

5. Polysaccharidmatrix nach einem der vorhergehenden Ansprüche, wobei das Polysaccharid aus der Gruppe, bestehend aus Celluloseestern, Celluloseethern, Cellulosehydrat, Agarose, Chitin, Chitosan, Dextran oder Kombinationen davon, ausgewählt ist.

6. Polysaccharidmatrix nach einem der vorhergehenden Ansprüche, wobei die mindestens eine ethylenische Monomerverbindung ein (Meth)Acrylsäurederivat ist.

7. Polysaccharidmatrix nach einem der vorhergehenden Ansprüche, wobei nach dem Pfropfen die Epoxid-Gruppen des auf die Polysaccharid-Ausgangsmatrix gepfropften Polymers mit zwei verschiedenen Liganden umgesetzt werden, von denen der erste eine Sulfonsäuregruppe und wobei der zweite Ligand Anilin oder 4-Aminobenzolsulfonsäure ist.

8. Verfahren zur Herstellung der Polysaccharidmatrix nach einem der vorhergehenden Ansprüche 1 bis 7, umfassend die Schritte:
A) Bereitstellen einer porösen Polysaccharid-Ausgangsmatrix, die mit einer organischen Säure behandelt ist,
wobei die organische Säure ausgewählt ist aus der Gruppe bestehend aus Citronensäure, Äpfelsäure, Weinsäure, Fumarsäure, Maleinsäure, Bernsteinsäure, Oxalsäure, Malonsäure, Ascorbinsäure, Glucuronsäure, Milchsäure oder Kombinationen davon,
und
B) Pfropfen der in Schritt A) bereitgestellten Matrix mit einem Gemisch, umfassend eine Übergangsmetall- oder Lanthanoidverbindung, ausgewählt aus der Gruppe der Cer-, Mangan-, Eisen-, Nickel-, Cobalt-, Vanadiumverbindungen oder Kombinationen davon, und mindestens eine ethylenische Monomerverbindung mit Epoxid-Gruppen zur Erzeugung eines auf die Polysaccharid-Ausgangsmatrix gepfropften Polymers mit Epoxid-Gruppen, wobei nach dem Schritt B) des Pfropfens die Epoxid-Gruppen des auf die Polysaccharid-Ausgangsmatrix gepfropften Polymers in einem anschließenden Schritt C) mit mindestens einem Liganden, der zur Wechselwirkung mit in Fluiden enthaltenen Adsorbenden befähigt ist und kationische und/oder anionische Gruppen aufweist, umgesetzt werden.

9. Verfahren zur Herstellung der Polysaccharidmatrix nach einem der vorhergehenden Ansprüche 1 bis 7, umfassend die Schritte:
A) Bereitstellen einer porösen Polysaccharid-Ausgangsmatrix, die mit einer Übergangsmetall- oder Lanthanoidverbindung behandelt ist, ausgewählt aus der Gruppe der Cer-, Mangan-, Eisen-, Nickel-, Cobalt-, Vanadiumverbindungen oder Kombinationen davon, und
B) Pfropfen der in Schritt A) bereitgestellten Matrix mit einem Gemisch, umfassend eine organische Säure, wobei die organische Säure ausgewählt ist aus der Gruppe bestehend aus Citronensäure, Äpfelsäure, Weinsäure, Fumarsäure, Maleinsäure, Bernsteinsäure, Oxalsäure, Malonsäure, Ascorbinsäure, Glucuronsäure, Milchsäure oder Kombinationen davon, und mindestens eine ethylenische Monomerverbindung mit Epoxid-Gruppen zur Erzeugung eines auf die Polysaccharid-Ausgangsmatrix gepfropften Polymers mit Epoxid-Gruppen, wobei nach dem Schritt B) des Pfropfens die Epoxid-Gruppen des auf die Polysaccharid-Ausgangsmatrix gepfropften Polymers in einem anschließenden Schritt C) mit mindestens einem Liganden, der zur Wechselwirkung mit in Fluiden enthaltenen Adsorbenden befähigt ist und kationische und/oder anionische Gruppen aufweist, umgesetzt werden.

10. Verfahren nach einem der Ansprüche 8 bis 9, wobei das Polysaccharid ausgewählt ist aus der Gruppe, bestehend aus Celluloseestern, Cellulosehydrat, Celluloseethern, Agarose, Chitin, Chitosan, Dextran und/oder Kombinationen davon.

11. Verfahren nach Anspruch 10, wobei das Polysaccharid ein Celluloseester ist und entweder nach Schritt B), während Schritt C) oder nach Schritt C) die Matrix zusätzlich mit einem basischen Medium behandelt wird.

12. Verwendung der Polysaccharidmatrix nach einem der Ansprüche 1 bis 7 zur Stofftrennung.

13. Verwendung der Polysaccharidmatrix nach Anspruch 12, wobei eine Polysaccharidmatrix nach Anspruch 7 als Kationenaustauscher zur Reinigung von Proteinen, Oligopeptiden, Polypeptiden oder Hormonen verwendet wird.

## Claims

1. Porous, non-particulate, convectively permeable polysaccharide matrix, on the surface of which a grafted polymer of at least one ethylene monomer compound is fixed, produced by grafting
- a porous, non-particulate, convectively permeable polysaccharide starting matrix together with
- the at least one ethylene monomer compound with epoxide groups in the presence of
- an organic acid, wherein the organic acid is selected from the group consisting of citric acid, malic acid, tartaric acid, fumaric acid, maleic acid, succinic acid, oxalic acid, malonic acid, ascorbic acid, glucuronic acid and lactic acid or combinations thereof, and
- a transition metal compound or lanthanide compound selected from the group of cerium compounds, manganese compounds, iron compounds, nickel compounds, cobalt compounds and vanadium compounds or combinations thereof for producing a polymer, which is grafted onto the polysaccharide starting matrix, with epoxide groups, wherein, after the grafting, the epoxide groups grafted onto the polysaccharide starting matrix are converted by at least one ligand which is capable of interaction with adsorbands contained in fluids and comprises cationic and/or anionic groups.

2. Polysaccharide matrix according to claim 1, wherein the polysaccharide starting matrix consists of polysaccharide.

3. Polysaccharide matrix according to claim 1, wherein the polysaccharide starting matrix is a porous support with a polysaccharide coating.

4. Polysaccharide matrix according to claim 3, wherein the porous support consists of a polymer selected from the group consisting of polyamide, poly(ether)sulphone, polyvinylidene difluoride, polyacrylnitrile, polyvinylchloride, polypropylene, polyethylene and polytetrafluoroethylene and their copolymers or mixtures thereof.

5. Polysaccharide matrix according to any one of the preceding claims, wherein the polysaccharide is selected from the group consisting of cellulose esters, cellulose ethers, cellulose hydrate, agarose, chitin, chitosan and dextran or combinations thereof.

6. Polysaccharide matrix according to any one of the preceding claims, wherein the at least ethylene monomer compound is a (meth)acrylic acid derivative.

7. Polysaccharide matrix according to any one of the preceding claims, wherein after the grafting the epoxide groups of the polymer grafted onto the polysaccharide starting matrix are converted by two different ligands, of which the first is a sulphonic acid group and wherein the second ligand is aniline or 4-aminobenzenesulphonic acid.

8. Method of producing the polysaccharide matrix according to any one of the preceding claims 1 to 7, comprising the steps of:
A) providing a porous polysaccharide starting matrix treated with an organic acid, wherein the organic acid is selected from the group consisting of citric acid, malic acid, tartaric acid, fumaric acid, maleic acid, succinic acid, oxalic acid, malonic acid, ascorbic acid, glucuronic acid and lactic acid or combinations thereof, and
B) grafting the matrix provided in step A) with a mixture comprising a transition metal compound or lanthanide compound selected from the group of cerium compounds, manganese compounds, iron compounds, nickel compounds, cobalt compounds and vanadium compounds or combinations thereof, and at least one ethylene monomer compound with epoxide groups for producing a polymer, which is grafted onto the polysaccharide starting matrix, with epoxide groups,
wherein, after the step B) of grafting, the epoxide groups of the polymer grafted onto the polysaccharide starting matrix are converted in a subsequent step C) by at least one ligand which is capable of interaction with adsorbands contained in fluids and comprises cationic and/or anionic groups.

9. Method of producing the polysaccharide matrix according to any one of the preceding claims 1 to 7, comprising the steps of:
A) providing a porous polysaccharide starting matrix treated with a transition metal compound or lanthanide compound selected from the group of cerium compounds, manganese compounds, iron compounds, nickel compounds, cobalt compounds and vanadium compounds or combinations thereof, and
B) grafting the matrix provided in step A) with a mixture comprising an organic acid, wherein the organic acid is selected from the group consisting of citric acid, malic acid, tartaric acid, fumaric acid, maleic acid, succinic acid, oxalic acid, malonic acid, ascorbic acid, glucuronic acid and lactic acid or combinations thereof, and at least one ethylene monomer compound with epoxide groups for producing a polymer, which is grafted onto the polysaccharide starting matrix, with epoxide groups,
wherein, after the step B) of grafting, the epoxide groups of the polymer grafted onto the polysaccharide starting matrix are converted in a subsequent step C) by at least one ligand which is capable of interaction with adsorbands contained in fluids and comprises cationic and/or anionic groups.

10. Method according to one of claims 8 and 9, wherein the polysaccharide is selected from the group consisting of cellulose esters, cellulose hydrate, cellulose ethers, agarose, chitin, chitosan and dextran and/or combinations thereof.

11. Method according to claim 10, wherein the polysaccharide is a cellulose ester and the matrix is additionally treated with a basic medium after step B), during step C) or after step C).

12. Use of the polysaccharide matrix according to any one of claims 1 to 7 for substance separation.

13. Use of the polysaccharide matrix according to claim 12, wherein a polysaccharide matrix according to claim 7 is used as a cation exchanger for purifying proteins, oligopeptides, polypeptides or hormones.

## Revendications

1. Matrice en polysaccharide poreuse non particulaire, perméable par convection, sur la surface de laquelle est fixé un polymère greffé constitué d'au moins un composé monomère éthylénique, fabriqué par greffage
- d'une matrice de départ en polysaccharide poreuse non particulaire, perméable par convection, avec
- ledit au moins un composé monomère éthylénique comportant des groupes époxyde
en présence
- d'un acide organique, qui est choisi dans le groupe constitué de l'acide citrique, de l'acide malique, de l'acide tartrique, de l'acide fumarique, de l'acide maléique, de l'acide succinique, de l'acide oxalique, de l'acide malonique, de l'acide ascorbique, de l'acide glucuronique, de l'acide lactique ou des combinaisons de ceux-ci, et
- d'un composé de métal de transition ou de lanthanoïde, choisi dans le groupe des composés du cérium, du manganèse, du fer, du nickel, du cobalt, du vanadium ou des combinaisons de ceux-ci,
pour la production d'un polymère greffé sur la matrice de départ en polysaccharide, comportant des groupes époxyde,
dans laquelle les groupes époxyde du polymère greffé sur la matrice de départ en polysaccharide sont mis à réagir, après le greffage, avec au moins un ligand qui est doué d'interaction avec des agents adsorbants contenus dans des fluides et présente des groupes cationiques et/ou anioniques.

2. Matrice en polysaccharide selon la revendication 1, dans laquelle la matrice de départ en polysaccharide se compose de polysaccharide.

3. Matrice en polysaccharide selon la revendication 1, dans laquelle la matrice de départ en polysaccharide est un support poreux doté d'un revêtement en polysaccharide.

4. Matrice en polysaccharide selon la revendication 3, dans laquelle le support poreux se compose d'un polymère choisi dans le groupe constitué du polyamide, de la poly(éther)sulfone, du difluorure de polyvinylidène, du polyacrylonitrile, du chlorure de polyvinyle, du polypropène, du polyéthène, du polytétrafluoroéthène, de leurs copolymères ou des mélanges de ceux-ci.

5. Matrice en polysaccharide selon l'une des revendications précédentes, dans laquelle le polysaccharide est choisi dans le groupe constitué des esters de cellulose, des éthers de cellulose, de l'hydrate de cellulose, de l'agarose, de la chitine, du chitosane, du dextrane ou des combinaisons de ceux-ci.

6. Matrice en polysaccharide selon l'une des revendications précédentes, dans laquelle ledit au moins un composé monomère éthylénique est un dérivé de l'acide (méth)acrylique.

7. Matrice en polysaccharide selon l'une des revendications précédentes, dans laquelle les groupes époxyde du polymère greffé sur la matrice de départ en polysaccharide sont mis à réagir, après le greffage, avec deux ligands différents dont le premier est un groupe acide sulfonique et le deuxième est l'aniline ou l'acide 4-aminobenzènesulfonique.

8. Procédé de fabrication de la matrice en polysaccharide selon l'une des revendications précédentes 1 à 7, comprenant les étapes de :
A) mise à disposition d'une matrice de départ en polysaccharide poreuse, qui est traitée avec un acide organique,
l'acide organique étant choisi dans le groupe constitué de l'acide citrique, de l'acide malique, de l'acide tartrique, de l'acide fumarique, de l'acide maléique, de l'acide succinique, de l'acide oxalique, de l'acide malonique, de l'acide ascorbique, de l'acide glucuronique, de l'acide lactique ou des combinaisons de ceux-ci,
et
B) greffage de la matrice mise à disposition à l'étape A) avec un mélange, comprenant un composé de métal de transition ou de lanthanoïde, choisi dans le groupe des composés du cérium, du manganèse, du fer, du nickel, du cobalt, du vanadium ou des combinaisons de ceux-ci, et au moins un composé monomère éthylénique comportant des groupes époxyde pour la production d'un polymère greffé sur la matrice de départ en polysaccharide, comportant des groupes époxyde,
dans lequel les groupes époxyde du polymère greffé sur la matrice de départ en polysaccharide étant mis à réagir, après l'étape B) de greffage, avec au moins un ligand qui est doué d'interaction avec des agents adsorbants contenus dans des fluides et présente des groupes cationiques et/ou anioniques, au cours d'une étape C) consécutive.

9. Procédé de fabrication de la matrice en polysaccharide selon l'une des revendications précédentes 1 à 7, comprenant les étapes de :
A) mise à disposition d'une matrice de départ en polysaccharide poreuse, qui est traitée avec un composé de métal de transition ou de lanthanoïde, choisi dans le groupe des composés du cérium, du manganèse, du fer, du nickel, du cobalt, du vanadium ou des combinaisons de ceux-ci, et
B) greffage de la matrice mise à disposition à l'étape A) avec un mélange, comprenant un acide organique, l'acide organique étant choisi dans le groupe constitué de l'acide citrique, de l'acide malique, de l'acide tartrique, de l'acide fumarique, de l'acide maléique, de l'acide succinique, de l'acide oxalique, de l'acide malonique, de l'acide ascorbique, de l'acide glucuronique, de l'acide lactique ou des combinaisons de ceux-ci, et au moins un composé monomère éthylénique comportant des groupes époxyde pour la production d'un polymère greffé sur la matrice de départ en polysaccharide, comportant des groupes époxyde,
dans lequel les groupes époxyde du polymère greffé sur la matrice de départ en polysaccharide sont mis à réagir, après l'étape B) de greffage, avec au moins un ligand qui est doué d'interaction avec des agents adsorbants contenus dans des fluides et présente des groupes cationiques et/ou anioniques, au cours d'une étape C) consécutive.

10. Procédé selon l'une des revendications 8 à 9, dans lequel le polysaccharide est choisi dans le groupe constitué des esters de cellulose, de l'hydrate de cellulose, des éthers de cellulose, de l'agarose, de la chitine, du chitosane, du dextrane et/ou des combinaisons de ceux-ci.

11. Procédé selon la revendication 10, dans lequel le polysaccharide est un ester de cellulose et la matrice est traitée en plus avec un milieu basique après l'étape B), pendant l'étape C) ou après l'étape C).

12. Utilisation de la matrice en polysaccharide selon l'une des revendications 1 à 7 pour la séparation de substances.

13. Utilisation de la matrice en polysaccharide selon la revendication 12, dans laquelle une matrice en polysaccharide selon, la revendication 7 est utilisée comme échangeuse de cations pour la purification de protéines, d'oligopeptides, de polypeptides ou d'hormones.
